(19) Europäisches Patentamt
European Patent Office
Office européen des brevets

(11) EP 0 484 754 B1

(12) EUROPÄISCHE PATENTSCHRIFT

(45) Veröffentlichungstag und Bekanntmachung des Hinweises auf die Patenterteilung:
**13.05.1998 Patentblatt 1998/20**

(51) Int Cl.$^6$: **C08G 77/58**, C08G 77/54, B01J 31/28

(21) Anmeldenummer: **91118159.2**

(22) Anmeldetag: **24.10.1991**

# (54) Geformte polymere Übergangsmetall-Komplexkatalysatoren mit Organosiloxan-Diphenylphosphinliganden

Shaped polymeric transition metal complex catalysts comprising organosiloxane-diphenylphosphine ligands

Catalyseurs polymériques granulaires du type de complexe de métaux de transition comprenant des ligands d'organosiloxane-diphénylphosphine

(84) Benannte Vertragsstaaten:
**AT BE CH DE DK ES FR GB IT LI LU NL**

(30) Priorität: **03.11.1990 DE 4035033**

(43) Veröffentlichungstag der Anmeldung:
**13.05.1992 Patentblatt 1992/20**

(73) Patentinhaber: **Degussa Aktiengesellschaft**
**60311 Frankturt (DE)**

(72) Erfinder:
- **Panster, Peter, Dr.**
  **W-6458 Rodenbach (DE)**
- **Gradl, Robert**
  **W-8755 Alzenau (DE)**
- **Kleinschmit, Peter, Dr.**
  **W-6450 Hanau 9 (DE)**

(56) Entgegenhaltungen:
**EP-A- 0 072 435**
**EP-A- 0 151 991**
**EP-A- 0 327 795**
**DE-A- 3 029 599**

Bemerkungen:
Die Akte enthält technische Angaben, die nach dem Eingang der Anmeldung eingereicht wurden und die nicht in dieser Patentschrift enthalten sind.

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 0 484 754 B1

Printed by Jouve, 75001 PARIS (FR)

## Beschreibung

Gegenstand der Erfindung sind polymere Übergangsmetall-Komplexkatalysatoren mit Organosiloxan-Diphenylphosphinliganden, die als geformte Copolykondensate vorliegen. Die geformten polymeren, unlöslichen Komplexverbindungen des Fe, Co, Ni, Ru, Rh, Pd, Os, Ir und/oder Pt weisen die verfahrens- und anwendungstechnischen Vorteile einer makroskopischen Kugelform auf und besitzen die für den Einsatz als heterogenisierter Komplexkatalysator erforderlichen physikalischen Eigenschaften. Gleichzeitig werden Verfahren beschrieben, nach denen die neuen Produkte nicht nur in der für den jeweiligen Einsatz gewünschten Kugelgröße, sondern auch mit den geeigneten physikalischen Eigenschaften hergestellt werden können. Weiterhin wird die Verwendung dieser polymeren Katalysatoren beschrieben.

Homogen eingesetzte Katalysatoren zeigen durchwegs eine höhere Aktivität und Selektivität als vergleichbare heterogen eingesetzte Katalysatoren. Größere verfahrenstechnische Schwierigkeiten bei ihrem Einsatz treten jedoch in der Regel im Zusammenhang mit ihrer Abtrennung vom gebildeten Produkt sowie vorhandenem Lösungsmittel und ihrer Recyclierung auf. Die Wiedergewinnung der teuren Edelmetallkomponente aus den Rückständen der Reaktionsmischung ist zudem aufwendig und normalerweise nur unter größeren Metallverlusten durchführbar.

Ein anderer Nachteil homogen eingesetzter Katalysatoren ist die häufig recht kurze Standzeit, verursacht durch die Bildung katalytisch inaktiver Spezies.

Um die genannten Nachteile sog. homogener Katalysatoren zu umgehen, wird bereits seit einiger Zeit weltweit die Entwicklung sog. heterogenisierter homogener Katalysatoren (heterogenisierter Katalysatoren) verfolgt, bei denen der normalerweise homogen eingesetzte Katalysator an einen festen Träger gebunden ist.

Der Stand der Technik auf diesem Gebiet der Katalyse ist bereits mehrfach in entsprechender Übersichtsliteratur zusammengefaßt worden, z. B. durch R.H. Grubbs in CHEMTECH Aug. 1977, S. 512, durch F.R. Hartley in "Catalysis By Metal Complexes", D. Reidel Publ. Comp., 1985, oder auch durch Yu. I. Yermakov et al. in "Catalysis By Supported Complexes", Elsivier Scientific Publ. Comp., 1981.

Die als Trägermaterialien eingesetzten organischen und anorganischen Polymersysteme erfüllen jedoch bisher aus unterschiedlichen Gründen die an sie gestellten Anforderungen nur sehr bedingt. Im Fall organischer Polymerträger stellen vor allem die physikalischen und mechanischen Eigenschaften sowie die zu geringe chemische Stabilität Schwachpunkte dar, während anorganische Polymerträger, wie Kieselgel, den Nachteil einer zu geringen und überdies mangelhaft definierten Funktionalisierbarkeit aufweisen.

Vor kurzem konnten nun, wie in der deutschen Patentschrift 30 29 599 beschrieben, neue heterogenisierte Metallkomplexkatalysatoren entwickelt werden, die die genannten Nachteile der bisherigen Systeme nicht aufweisen. Die Matrix dieser Polysiloxankatalysatoren besitzt praktisch die Vorzüge eines anorganischen Polymerträgers und läßt sich darüber hinaus quasi maßgeschneidert herstellen, z. B. in bezug auf die wichtigen Aspekte, daß das Metall : Ligand-Verhältnis variiert werden kann oder sogenannte Vernetzer in die Matrix integriert werden können oder eine Steuerung der Katalysezentrendichte und -verteilung möglich ist. Gegenüber Systemen mit rein anorganischen Trägern weisen diese Organopolysiloxan-Polymeren vor allem die Vorteile einer höheren Metallkonzentration, der einfacheren präparativen Zugänglichkeit und der höheren Stabilität gegenüber einem chemischen Abbau auf.

Nach diesem Konzept wurden insbesondere die in der deutschen Patentschrift 30 29 599 erwähnten polymeren Metall-Phosphinkomplexe synthetisiert, die generell sehr gute katalytische Eigenschaften aufweisen. Diese heterogenisierten Komplexkatalysatoren haben jedoch den Nachteil, daß sie bisher nur in relativ undefinierter makroskopischer Gestalt und nicht in der anwendungstechnisch günstigen Kugelform mit den gewünschten physikalischen und morphologischen Eigenschaften hergestellt werden konnten.

In ebenso undefinierter makroskopischer Gestalt lassen sich gemäß EP-A - 0 072 435 polymere Metall-Amin-Komplexverbindungen herstellen.

Geformte (kugelförmige) polymere tertiäre oder sekundäre Organosiloxanaminverbindungen sind dagegen aus der EP-A- 0 327 795 bekannt. Diese weisen jedoch aufgrund des Herstellverfahrens keinen Gehalt an Übergangsmetallen auf. Dieser kann erst durch einen weiteren Adsorptionsschritt erzeugt werden.

Aufgabe der vorliegenden Erfindung ist daher, heterogenisierte Übergangsmetall-Komplexe mit Organosiloxan-Diphenylphosphinliganden in Kugelform und mit den gewünschten physikalischen Eigenschaften direkt und reproduzierbar herzustellen.

Gegenstand der Erfindung sind geformte polymere Metallkomplexe des Eisens, Kobalts, Nickels, Rutheniums, Rhodiums, Palladiums, Osmiums, Iridiums und/oder Platins. Sie sind dadurch gekennzeichnet, daß der Ligand aus einem geformten Organosiloxan-Copolykondensat, das aus Einheiten der Formel

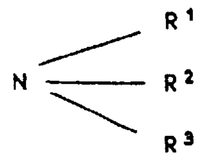

(I)

und aus Einheiten der Formel

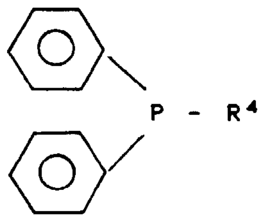

(II)

besteht, wobei das jeweilige Zentralatom über die bindungsstarken Phosphoratome der Phosphin-Einheiten (II) oder zusätzlich auch über die bindungsschwächeren Stickstoffatome der Amineinheiten (I) koordinativ gebunden ist, $R^2$ bis $R^4$ gleich oder verschieden sind und eine Gruppe der allgemeinen Formel

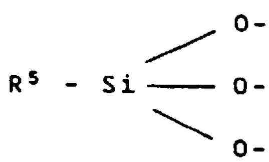

(III)

bedeuten, wobei $R^5$ direkt an das Phosphoratom bzw. an das Stickstoffatom gebunden ist und eine lineare oder verzweigte Alkylengruppe mit 1 bis 10 C-Atomen, eine Cyloalkylengruppe mit 5 bis 8 C-Atomen oder eine Einheit der allgemeinen Formel

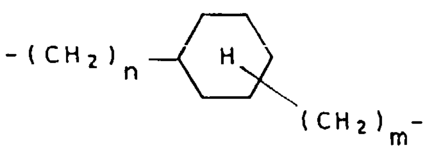

oder

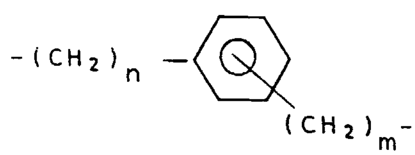

darstellt, in der n und m eine Zahl von 0 bis 6 ist, wobei n die Zahl N-ständiger bzw. P-ständiger und m die Zahl Si-ständiger Methylengruppen angibt, $R^1$ ebenfalls eine Gruppe der Formel (III) darstellt oder für H, $CH_3$, $C_2H_5$, $C_3H_7$ steht, wobei die freien Valenzen der an das Si-Atom gebundenen Sauerstoffatome wie bei Kieselsäuregerüsten durch Siliciumatome weiterer Gruppen der Formel (III) und/oder über die Metallatome in einem oder mehreren vernetzenden Brückengliedern

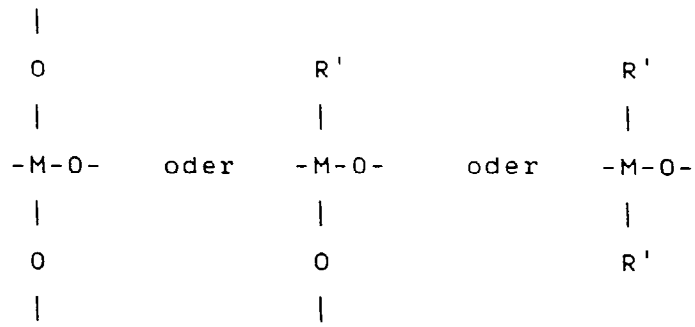

(IV)

bzw.

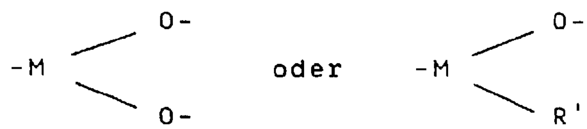

mit M = Al, abgesättigt sind, M ein Si-, Ti-, oder Zr-Atom und R' eine lineare oder verzweigte Alkylgruppe mit 1 bis 5 C-Atomen oder eine Phenylgruppe ist und das Verhältnis der Siliciumatome aus den Gruppen der allgemeinen Formel (III) zu den Metallatomen in den vernetzenden Brückengliedern (IV) 1 : 0 bis 1 : 20 und das molare Verhältnis an Phosphin-Einheiten (II) zu komplexierten Metalleinheiten 1 : 1 bis 1000 : 1, vorzugsweise 1 : 1 bis 100 : 1, beträgt und die polymeren Komplexkatalysatoren makroskopisch als kugelförmige Teilchen mit einem Durchmesser von 0,01 bis 3,0 mm, vorzugsweise 0,05 bis 2,0 mm, einer spezifischen Oberfläche von > 0 bis 1000 $m^2/g$, vorzugsweise > 0 bis 700 $m^2/g$, einem spezifischen Porenvolumen von 0.01 bis 6,5 ml/g, sowie einer Schüttdichte von 50 bis 1000 g/l, vorzugsweise 100 bis 700 g/l, vorliegen.

Im Rahmen der Ausarbeitung der Erfindung erwies es sich als besonders vorteilhaft, sowohl in bezug auf die Herstellung und die physikalischen Eigenschaften als auch im Hinblick auf die katalytischen Eigenschaften der heterogenisierten Komplexkatalysatoren als Polymerligandsystem ein Copolykondensat mit Amin- und Phosphingruppen zu verwenden. Derartige geformte Copolykondensate sind prinzipiell bereits in der deutschen Patentanmeldung P 39 25 359.7 beschrieben.

Das Verhältnis von Einheiten nach Formel (I) zu Einheiten nach Formel (II) ist stark variierbar und kann innerhalb der Grenzen 10: 90 bis 95 : 5 Mol% liegen. Probleme mit den morphologischen, physikalischen und chemischen Eigenschaften der erfindungsgemäßen polymeren Komplexkatalysatoren treten dabei nicht auf.

Eine besondere Ausführungsform der Erfindung sieht vor, daß $R^1$ bis $R^4$ eine Gruppe der allgemeinen Formel (III) sind und gleich oder verschieden sind.

Das in der Praxis zu wählende Verhältnis hängt primär von dem herzustellenden Komplex sowie dem vorgesehenen Einsatzgebiet und den hierfür erforderlichen chemischen und physikalischen Eigenschaften ab, z. B. davon, ob eine hohe Metallkonzentration oder eine hohe Dichte an der Phosphin- oder Aminkomponente im Hinblick auf katalytische Eigenschaften oder auf Metallhaftung erforderlich ist oder nicht.

Die monomeren Bausteine des geformten Polymerligandsystems sind prinzipiell bekannte Verbindungen, beispielsweise der Formeln

$N[(CH_2)_3Si(OC_2H_5)_3]_3$
$N[(CH_2)_{10}Si(OCH_3)_3]_3$
$(C_6H_5)_2P(CH_2)_3Si(OCH_3)_3$
$Si(OC_2H_5)_4$, $(H_3C)_2Si(OC_2H_5)_2$
$Ti(OC_3H_7)_4$

Die Zusammensetzung der daraus erhältlichen Polymereinheiten läßt sich durch die Formeln

$N[(CH_2)_3SiO_{3/2}]_3$
$N[(CH_2)_{10}SiO_{3/2}]_3$
$(C_6H_5)_2P(CH_2)_3SiO_{3/2}$
$SiO_{4/2}$, $(H_3C)_2SiO_{2/2}$
$TiO_{4/2}$

beschreiben.

Die geformten Copolykondensate können selbst bei gleicher chemischer Zusammensetzung in völlig unterschiedlicher Form als sog. statistische Copolykondensate ("Random-Copolykondensate") oder als Block-Copolykondensate oder auch als sog. gemischte Copolykondensate vorliegen. Erfindungsgemäß können die geformten Polymerligandsysteme in bezug auf die Einheiten nach Formeln (I), (II) und (IV) in jeder der drei genannten Formen vorliegen. Dies bedeutet, daß im Falle eines rein statistischen Copolykondensates, das Einheiten nach Formel (I) und (II) und gegebenenfalls (IV) enthält, eine statistische Verteilung der Komponenten entsprechend der molaren Verhältnisse der Ausgangsprodukte unter Berücksichtigung der im Fall der Einheiten (I) und (II) jeweils vorhandenen Siliciumgruppierungen nach Formel (III) und der Funktionalität der Vernetzergruppierung (IV) gegeben ist. Im Falle eines sog. Block-Copolykondensates liegt eine Bildung von Blöcken gleicher Einheiten nach Formel (I) und (II) und gegebenenfalls (IV) vor. Schließlich weist ein sog. gemischtes Copolykondensat sowohl Strukturen eines statistischen Copolykondensates als auch eines Block-Copolykondensates auf. Dabei können die Einheiten nach Formel (I) oder Formel (II) oder Formel (IV) sowohl als statistisches als auch als Block-Copolykondensat vorliegen.

Besondere Vorteile bezüglich der Verfügbarkeit der Ausgangsmaterialien und der stofflichen Eigenschaften werden mit Polymerligandsystemen erreicht, bei denen $R^1$ bis $R^4$ für eine Gruppe der allgemeinen Formel

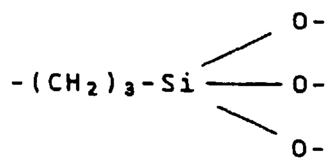

(V)

stehen.

Bei den bevorzugten metallhaltigen Gruppen, die an die Polymereinheiten nach Formel (II) und Formel (I) komplex gebunden sind, handelt es sich um eine oder mehrere Metalleinheiten (VI) von

$FeX_3$, $FeX_2$
$CoX_3$, $CoX_2$,
$NiX_2$
$RuX_3$, $RuX_2$,
$RhX_3$, $RhX_2$, RhX, Rh(dien)X, RhX(CO)
$PdX_4$, $PdX_2$, $Pd^o$
$OsX_3$
$IrX_3$, IrX, Ir(dien)X, IrX(CO)
$PtX_4$, $PtX_2$, $Pt^o$,

wobei X für Cl, Br, J, H, Acetylacetonat, Acetat, 0.5 $SO_4$, $NO_3$, CN und dien für Cyclooctadien, Norbornadien steht.

Die durch Komplexbildung zwischen diesen Metalleinheiten und dem Polymerligandsystem gebildeten Komplexstrukturen sind prinzipiell aus der Komplexchemie dieser Metalle bekannt und dem Komplexchemiker vertraut (vgl. z. B. die Buchserie "Inorganic Syntheses", John Wiley & Sons, New York, Chichester, Brisbane, Toronto, Singapore oder Inorganic Chemistry of the Transition Elements, Chemical Society, Burlington House, London W1V OBN).

Sie lassen sich für die einzelnen erfindungsrelevanten Metalle z. B. durch folgende Formeln beschreiben:

$FeX_3L_3$, $FeX_2L_4$
$CoX_3L_2$, $CoX_3L_3$, $CoX_2L_3$, $CoX_2L_4$
$NiX_2L_2$, $NiL_4$
$RuX_3L_3$
$RhX_3L_3$, $RhX_2L_3$, $RhXL_3$, $RhL_4^+X^-$
$PdX_4L_2$, $PdX_2L_2$, $PdL_4$
$OsX_3L_3$

$IrX_3L_3$, $IrXL_3$
$PtX_4L_2$, $PtX_2L_2$, $PtL_4$

X = Cl, Br, J, H, Acetylacetonat, Acetat, $\frac{1}{2}$ $SO_4$, $NO_3$, CN

L = Ligand

Die aus der Komplexchemie dieser Metalle bekannten löslichen Komplexstrukturen lassen sich natürlich auch auf die Polymerligand-gebundenen unlöslichen Metalleinheiten übertragen. Dies bedeutet im Fall der erfindungsgemäßen geformten Übergangsmetall-Komplexkatalysatoren, daß L eine Polymerligandeinheit der Formel (I) oder Formel (II) darstellt, welche die Ankergruppen darstellen, über die die vorstehend genannten Metalleinheiten an die Polymermatrix gebunden sind.

Im Fall der erfindungsgemäßen heterogenisierten Komplexkatalysatoren ist für die katalytischen Eigenschaften günstig, wenn die vorstehend genannten Metalleinheiten nach Formel (VI) jeweils über mindestens eine Phosphineinheit nach Formel (II) an die Polymermatrix gebunden sind.

Eine bevorzugte Ausführungsform der Erfindung sieht vor, daß die Metalleinheiten nach Formel (VI) jeweils nur über Phosphineinheiten nach Formel (II) an die Polymermatrix gebunden sind.

Für die Praxis ist vorteilhaft, wenn der Metallgehalt im Polymersystem mindestens 0,01 Gew.% und höchstens 18 Gew.% beträgt. Besonders bevorzugt werden Metallgehalte im Polymersystem von mindestens 0,1 Gew.% und höchstens 10 Gew.%.

Im Hinblick auf die katalytischen Eigenschaften und die Metallhaftung der erfindungsgemäßen Verbindungen sind die Phosphineinheiten nach Formel (II) die entscheidenden Ligandenkomponenten bei dem Aufbau der polymeren Metallmatrix-Verbindung, während die Amingruppierungen vor allem vorteilhafte physikalische Eigenschaften und zum Teil auch chemische Eigenschaften des Polymers gewährleisten.

Die Zusammensetzung der Verbindungen gemäß der Erfindung kann über bestimmte Herstellungsmaßnahmen, die daraus resultierende Verteilung der beiden Ligandentypen nach Formel (I) und (II) und deren stöchiometrisches Verhältnis beeinflußt werden. Grundsätzlich ist natürlich aus der Komplexchemie bekannt, daß ein Phosphinligand vom Typ der Ligandeneinheit nach Formel (II) (Typ: Diphenylalkylphosphin) ein wesentlich stärkeres Komplexierungsvermögen besitzt als ein Aminligand vom Typ der Ligandeinheit nach Formel (I). Dieser Tatsache ist bei der Konzeption der aufzubauenden polymeren Metallkomplexe und der Wahl der Maßnahmen Rechnung zu tragen, denn in der Regel wird im Fall einer Konkurrenzsituation stets vorrangig der Phosphinligand das Zentralatom des Übergangsmetalls komplexieren.

Die angegebenen Metallkonzentrationen berücksichtigen die Tatsache, daß neben den die fixierten Metallzentren nach Formel (VI) komplexierenden Liganden nach Formeln (II) und (I) noch weitere überschüssige und nicht komplexierende Liganden nach Formeln (I) und/oder (II) im Polymersystem vorhanden sind. Eine besondere Ausführungsform der Erfindung sieht vor, daß im Polymersystem nicht mehr Ligandeinheiten nach Formel (II) vorliegen, als zum Aufbau des jeweiligen Metallkomplexes maximal benötigt werden, so daß das stöchiometrische Verhältnis zwischen den Liganden nach Formel (II) und dem Metall mindestens 1 : 1 aber, in Abhängigkeit vom jeweiligen Metall für Fe, Co, Rh, Pd, Pt, Ni maximal 4 : 1 und für Ru, Os, Ir maximal 3 : 1 beträgt und daneben weitere Liganden nach Formel (I) im Polymersystem vorliegen. Im Fall eines Verhältnisses von 1 : 1 müssen natürlich auch Amineinheiten nach Formel (I) zum Aufbau des polymeren Metallkomplexes herangezogen werden.

Bei einer Reihe von polymeren Katalysatoren kann es in Abhängigkeit von der Art der zu katalysierenden Reaktion, z. B. im Hinblick auf die Erzielung einer verbesserten Metallhaftung oder verbesserter Selektivitätseigenschaften, vorteilhaft sein, wenn auch überschüssige Polymerligandeinheiten nach Formel (II) über das Verhältnis von 4 : 1 bzw. 3 : 1 hinaus in der Polymermatrix vorhanden sind. Auch diese überschüssigen Liganden nach Formel (II) können in bezug auf die Amineinheiten nach Formel (I) und die gegebenenfalls vorhandenen Vernetzer sowohl als statistische, Block- oder gemischte Copolykondensate vorliegen.

Insgesamt sind die Extremwerte der denkbaren Zusammensetzungen einerseits durch die Grenzwerte des molaren Verhältnisses der Einheiten nach Formel (I) zu den Einheiten nach Formel (II) von 95 : 5 Mol% bis 10 : 90 Mol% und andererseits durch die möglichen Metallgehalte von 0,01 bis zu 18 Gew.% vorgegeben.

Gegenstand der Erfindung sind auch Verfahren zur Herstellung der erfindungsgemäßen geformten polymeren Übergangsmetall-Komplexkatalysatoren. Verwendung finden dabei nahezu ausschließlich Metallausgangsverbindungen, die präparativ relativ leicht zugänglich sind und kommerziell verfügbar sind. Die der Polykondensationsstufe, d. h. der Ausbildung der Polymermatrix, vorangehende Präparation des Monomerkomplexes unter Verwendung von Siliciumsubstituierten Monomerliganden der allgemeinen Formel

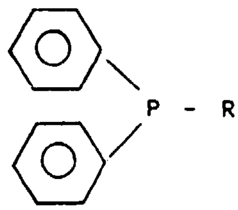

und gegebenenfalls der allgemeinen Formel

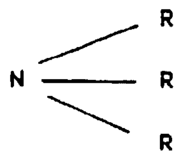

erfolgt bei diesen erfindungsgemäßen Verfahren nach bekannten Prinzipien der Übergangsmetallchemie, wie sie z. B. in der vorstehend zitierten Literatur oder in wissenschaftlichen Veröffentlichungen über die Komplexchemie der hier genannten Metalle in allgemeiner Form beschrieben sind.

Ein erstes Verfahren zur Herstellung der geformten polymeren Metallkomplexe ist dadurch gekennzeichnet, daß man eine oder mehrere wasserhaltige oder wasserfreie Metallverbindungen (VII) von

$FeX_3$, $FeX_2$
$CoX_3$, $CoX_2$
$NiX_2$
$RuX_3$, $RuX_3(CH_3CN)_3$, $RuX_3(C_6H_5CN)_3$
$Y_3RhX_6$, $RhX_3$, $RhX_3(CH_3CN)_3$,
$RhX_3(C_6H_5CN)_3$, $RhX_2$, $[RhX(dien)]_2$
$Y_2PdX_6$, $Y_2PdX_4$, $PdX_2$
$OsX_3$, $OsX_3(CH_3CN)_3$, $OsX_3(C_6H_5CN)_3$
$Y_3IrX_6$, $IrX_3$, $IrX_3(CH_3CN)_3$,
$IrX_3(C_6H_5CN)_3$, $[IrX(dien)]_2$
$Y_2PtX_6$, $M_2PtX_4$, $PtX_2$,

wobei

| | |
|---|---|
| X = | Cl, Br, J, Acetylacetonat, Acetat, $\frac{1}{2}$ $SO_4$, $NO_3$, CN |
| dien = | Cyclooctadien, Norbornadien und |
| Y = | H, Na, K, $NH_4$ |

in einem Lösungsmittel oder Lösungsmittelgemisch mit vorzugsweise polarer Natur, gegebenenfalls bei erhöhter Temperatur, über einen Zeitraum von 1 Min. bis zu 48 Stunden mit einem Phosphin der allgemeinen Formel

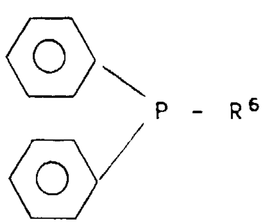

(VIII)

wobei $R^6$ eine Gruppe der allgemeinen Formel

$$R^5 - Si(OR^7)_3 \quad (IX)$$

bedeuten, $R^5$ dieselbe Bedeutung wie in Formel (III) von Anspruch 1 hat, $R^7$ eine lineare oder verzweigte Alkylgruppe mit 1 bis 5 C-Atomen bedeutet und das Verhältnis zwischen der Molzahl an Phosphin nach Formel (VIII) und der Molzahl der insgesamt komplex gebundenen Metallatome in den Metallverbindungen nach Formel (VII) wenigstens 1 : 1 bis 1000 : 1, vorzugsweise 1 : 1 bis 100 : 1 beträgt, zum Metallkomplex umsetzt, der erhaltenen Lösung anschließend ein Aminosilan der allgemeinen Formel

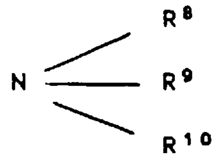

(X)

wobei $R^8$ für H, $CH_3$, $C_2H_5$, $C_3H_7$ oder eine Gruppe der allgemeinen Formel (IX) und $R^9$ sowie $R^{10}$ ebenfalls für eine Gruppe der Formel (IX) stehen, in der $R^5$ und $R^7$ denselben Bedeutungsumfang wie in Formel (IX) haben sowie gegebenenfalls eine oder mehrere Verbindungen der allgemeinen Formel

$$M(OR)_{2\text{-}4}\, R'_{0\text{-}2} \text{ bzw. } M(OR)_{2\text{-}3}\, R'_{0\text{-}1} \quad (XI)$$

wobei M ein Si-, Ti-, Zr- oder Al-Atom, R' eine lineare oder verzweigte Alkylgruppe mit 1 bis 5 C-Atomen oder eine Phenylgruppe ist, R eine lineare oder verzweigte Alkylgruppe mit 1 bis 5 C-Atomen bedeutet und das Verhältnis der Siliciumatome aus den Gruppen der allgemeinen Formel (IX) zu den Metallatomen in den Vernetzern (XI) 1 : 0 bis 1 : 20 beträgt, zusetzt, dann der erhaltenen Lösung unter Rühren eine zumindest für die vollständige Hydrolyse und Kondensation ausreichende Menge Wasser zufügt, das Reaktionsgemisch über einen Zeitraum bis zu 6 Stunden, bevorzugt bei Rückflußtemperatur, hydrolysiert, dann unter Weiterrühren bei einer bestimmten Temperatur im Bereich von Raumtemperatur bis 200° C unter der Maßgabe gelieren läßt, daß man es bei Gelierungsbeginn oder bis zu einer Stunde danach mit 10 bis 2000, vorzugsweise 50 bis 500 Gew.%, bezogen auf die Gesamtmenge von Phosphin (VIII), Aminoorganosilan (X) und gegebenenfalls Vernetzer (XI), eines weitgehend wasserunlöslichen, aber die (an)gelierte Reaktionsmischung lösenden Lösungsmittels versetzt und homogenisiert, dem viskosen Homogenisat sofort oder im Zeitraum bis zu 10 Stunden, gegebenenfalls unter Erhöhung der ursprünglich eingestellten Temperatur, 10 bis 2000, vorzugsweise 50 bis 500 Gew.%, bezogen auf die Gesamtmenge von Phosphin (VIII), Aminoorganosilan (X) und gegebenenfalls Vernetzer (XI), Wasser zugibt, die den monomeren Metallkomplex enthaltende organische Phase in dem flüssigen Zweiphasensystem dispergiert und den sich in Form von Kugeln bildenden Feststoff nach dafür ausreichender Reaktionszeit bei einer Temperatur von Raumtemperatur bis 200° C von der flüssigen Phase abtrennt, dann gegebenenfalls mit einem niedrigsiedenden Lösungsmittel extrahiert, bei Raumtemperatur bis 250° C, gegebenenfalls unter Schutzgas oder im Vakuum trocknet und 1 bis 100 Stunden bei Temperaturen von 150° C bis 300° C tempert und/oder klassifiziert.

Nach dieser ersten Verfahrensweise werden in Abhängigkeit von der Stöchiometrie in bezug auf alle vorhandenen Polymereinheiten nach Formel (I) und (II) sowie gegebenenfalls vorhandene Gruppen nach Formel (IV) gemischte oder statistische Copolykondensate erhalten. Zu beachten ist, daß durch die Komplexierung der Phosphineinheiten nach Formel (II) am Metallzentrum eine Blockbildung erfolgt und bei Verwendung wasserhaltiger Metallverbindungen (VII) eine teilweise Vorkondensation der zugesetzten monomeren Phosphine nach Formel (VIII) bereits während deren Umsetzung mit der Metallkomponente stattfindet. Bei Verwendung wasserfreier Metallverbindungen (VII) ist jedoch für gegebenenfalls über die Höchstkoordinationszahl hinaus vorhandene Phosphineinheiten nach Formel (II) und in bezug auf nicht oder wenig komplexierende Aminliganden (I) sowie gegebenenfalls vorhandene Vernetzergruppen (IV) von der Bildung einer statistischen Verteilung auszugehen.

Prinzipiell können als Ausgangsverbindungen für das Verfahren anstelle der Alkoxysilylverbindungen auch die entsprechenden Halogenid- oder Phenoxyverbindungen eingesetzt werden, doch bietet deren Verwendung keine Vorteile, sondern kann z. B. im Fall der Chloride, Schwierigkeiten durch die bei der Hydrolyse freiwerdende Salzsäure verursachen.

Die Hydrolyse von Ausgangsstoffen und gegebenenfalls Vernetzer(n) muß in einem weitgehend wassermischbaren, aber die Ausgangsstoffe lösenden Lösungsmittel durchgeführt werden. Bevorzugt werden dabei Alkohole verwendet, die zu den Alkoxygruppierungen an den monomeren Vorstufen der Ausgangsstoffe bzw. an den Metallatomen der gegebenenfalls eingesetzten Vernetzer korrespondieren.

Besonders geeignet sind Methanol, Ethanol, n- und i-Propanol, n- und i-Butanol oder n-Pentanol. Auch Gemische solcher Alkohole können angewandt werden. Anstelle von Alkoholen können auch andere polare Lösungsmittel, die weitgehend wassermischbar sind, eingesetzt werden, doch erweist sich dies aus verfahrenstechnischen Gründen wegen der mit dem hydrolytisch abgespaltenen Alkohol zustandekommenden Lösungsmittelgemische als wenig sinnvoll.

Bevorzugt führt man die Hydrolyse mit einem Überschuß an Wasser über die stöchiometrisch erforderliche Menge durch. Die zur Hydrolyse benötigte Menge Wasser hängt von der Hydrolysegeschwindigkeit des verwendeten Phosphins (VIII), Amins (X) und Vernetzers (XI) derart ab, daß mit zunehmender Menge Wasser raschere Hydrolyse erfolgt; allerdings kann eine Obergrenze durch auftretende Entmischung und Ausbildung eines Zweiphasensystems vorgegeben sein. Aufgrund der genannten beiden Aspekte wird in praxi gewichtsmäßig etwas weniger Wasser verwendet als Organosilane zuzüglich Vernetzer. Die Dauer der Hydrolyse hängt von der Hydrolyseneigung der Ausgangsstoffe und/oder Vernetzer und von der Temperatur ab. Die Hydrolysebereitschaft und damit -geschwindigkeit hängt insbesondere von der Art der Silicium- bzw. Titan-, Zirkonium- und Aluminium-ständigen Alkoxygruppen ab, wobei die Methoxygruppe am schnellsten hydrolysiert. Daneben hängt die Dauer des Gesamtvorganges Hydrolyse und Polykondensation auch von der Basizität des Aminoorganosilans ab. Amine fungieren bekanntlich als Kondensationsbeschleuniger, so daß sie eine Autokatalyse bewirken können.

Hydrolyse und Polykondensation werden generell durch Zusatz von Basen, vorzugsweise von Ammoniak, oder von anorganischen oder organischen Säuren, aber auch durch das katalytisch aktive Metall selbst, oder durch Zusatz von üblichen Kondensationskatalysatoren, wie z. B. Dibutylzinndiacetat, beschleunigt.

Das Erfordernis, den in Lösungsmittel gelösten und mit Wasser versetzten Ausgangsstoff unter Weiterrühren auf einer bestimmten Temperatur zu halten, resultiert daher, daß die Geschwindigkeit der sich durch Gelierung anzeigenden Polykondensation temperaturabhängig ist.

Die in der Hydrolyse- bzw. Gelierphase anzuwendende Temperatur wird im Einzelfall empirisch ermittelt und festgelegt. Sie ist so zu wählen, daß im darauffolgenden Verfahrensschritt, der sog. Formungsphase, eine gelartige Masse erhalten bleibt.

Die mit der Überführung der kohärenten, metallhaltigen, flüssigkeitsdurchsetzten gelartigen Masse in separate sphärische Teilchen einhergehende Formungsphase beginnt mit dem Versetzen der (an)gelierten Reaktionsmischung mit einem weitgehend wasserunlöslichen, aber die Reaktionsmischung ausreichend lösenden Lösungsmittel in der vorgesehenen Menge.

Geeignete Lösungsmittel sind z. B. lineare oder verzweigte Alkohole mit 4 bis 18 C-Atomen oder Phenole, lineare oder verzweigte symmetrische oder unsymmetrische Dialkylether sowie Di- oder Triether (wie Ethylenglycoldimethylether), chlorierte oder fluorierte Kohlenwasserstoffe, mit einer oder mehreren Alkylgruppen substituierte Aromaten oder Aromatengemische, wie z. B. Toluol oder Xylol, weitgehend mit Wasser nicht mischbare symmetrische oder unsymmetrische Ketone.

Bevorzugt werden der (an)gelierten Reaktionsmischung jedoch ein linearer oder verzweigter Alkohol mit 4 bis 12 C-Atomen, Toluol, Ethylbenzol oder o-, m-, p-Xylol, oder Gemische davon, zugesetzt.

Dieser Lösungsmittelzusatz bewirkt nach der Homogenisierung mit der Reaktionsmischung eine Verdünnung und damit eine deutliche Verlangsamung der mit Viskositätszunahme einhergehenden Kondensationsreaktion.

Die Bemessung der Menge dieses in der Formungsphase verwendeten Lösungsmittels hängt insbesondere davon ab, welche Korngröße jeweils für den geformten polymeren Übergangsmetall-Komplexkatalysator angestrebt wird. Als Faustregel kann gelten, daß für grobes Korn (= Kugeln von größerem Durchmesser) wenig, für feines Korn (Kugeln von kleinerem Durchmesser) viel Lösungsmittel zu verwenden ist. Einfluß auf die Korngröße nimmt darüber hinaus auch die Intensität, mit der das viskose Homogenisat aus dem sich bildenden Reaktionsprodukt und dem weitgehend wasserunlöslichen Lösungsmittel in der Wasserphase dispergiert wird. Durch starkes Rühren wird die Ausbildung eines feineren Kornes begünstigt. Zur Stabilisierung der wäßrigen Dispersion der Siloxan enthaltenden organischen Phase kann eines der bekannten Dispergierhilfsmittel, wie langkettige Carbonsäuren oder deren Salze oder Polyalkylenglycole, in üblichen Konzentrationen, zugesetzt werden.

Die bevorzugte Temperatur, bei der die Dispergierung der Siloxan enthaltenden organischen Phase in der wäßrigen Phase durchgeführt und aus der dispersen Phase kugelförmiger Feststoff gebildet wird, ist in der Regel die Rückflußtemperatur der Gesamtmischung. Grundsätzlich können aber dieselben Temperaturen wie in der Gelierstufe angewandt werden. Die Gesamtdauer von Dispergierungsstufe und Nachreaktion beträgt in der Regel 0,5 bis 10 Stunden.

Sowohl die Gelierung als auch die Formung können bei Normaldruck oder einem Überdruck, welcher der Summe der Partialdrucke der Komponenten der Reaktionsmischung bei der jeweils angewandten Temperatur entspricht, durchgeführt werden.

Die Abtrennung des kugelig geformten feuchten Produktes von dem flüssigen Dispersionsmittel kann durch übliche Maßnahmen, wie Dekantieren, Abfiltrieren oder Zentrifugieren erfolgen. Man kann dazu aber auch die flüssige Phase

aus dem Reaktor entfernen, den verbleibenden Feststoff darin ein- oder mehrmals mit einem niedrigsiedenden Extraktionsmittel, bevorzugt einem niedrigsiedenden Alkohol, behandeln, um durch zumindest teilweisen Austausch des meist relativ hochsiedenden Lösungsmittels der Formungsphase gegen das niedrigsiedende Extraktionsmittel die spätere Trocknung des geformten Katalysators zu erleichtern.

Die Trocknung kann grundsätzlich bei Raumtemperatur bis 250° C, gegebenenfalls unter Schutzgas oder im Vakuum, durchgeführt werden. Zur Härtung und Stabilisierung kann der getrocknete geformte Feststoff bei Temperaturen von 150 bis 300° C getempert werden.

Das getrocknete bzw. getemperte Produkt kann in üblichen Vorrichtungen in verschiedene Korngrößenfraktionen klassifiziert werden. Von den Aufarbeitungsmaßnahmen Extraktion, Trocknung, Temperung und Klassifizierung kann, je nach den Umständen, die eine oder andere entfallen. Eine Klassifizierung kann an flüssigkeitsfeuchtem, getrockneten oder getemperten Produkt durchgeführt werden.

Nach einer Variante des erfindungsgemäßen Verfahrens wird ein Teil oder auch die Gesamtmenge des bei oder nach Gelierungsbeginn zuzusetzenden, weitgehend wasserunlöslichen Lösungsmittels schon in der Hydrolysestufe neben dem dort verwendeten Lösungsmittel der Reaktionsmischung zugesetzt. Bei Teilzugabe wird der Rest nach Gelierungsbeginn zugesetzt. Im Extremfall der Zugabe der Gesamtmenge kann das Dispersionsmittel Wasser bei oder nach Gelierungsbeginn zugesetzt werden. Diese Variante wird dann bevorzugt angewendet, wenn das Gemisch aus dem hergestellten Si-substituierten Monomerkomplex und gegebenenfalls vorhandenem überschüssigen Phosphin nach Formel (VIII) und Amin (X) sowie gegebenenfalls Vernetzer (XI) eine außerordentlich hohe Hydrolyse- und Polykondensationsneigung zeigt.

Im Hinblick auf die Einstellung und Fixierung einer bestimmten definierten Ligandensphäre um das polymergebundene Metallzentrum kann es besonders vorteilhaft sein, wenn man nach einer Variante der vorstehend beschriebenen Verfahrensweise den nach Umsetzung mit dem Phosphin nach Formel (VIII) mit der Metallverbindung nach Formel (VII) erhaltenen monomeren Phosphinkomplex und den gegebenenfalls noch im Gemisch vorhandenen, nicht zur Komplexbildung benötigten überschüssigen Phosphinanteil nach Formel (VIII), bis zu dem maximalen Verhältnis Phosphin (VIII) zu Metallverbindung (VII) von 1000 : 1, gegebenenfalls nach Zusatz einer oder mehrerer der Verbindungen der allgemeinen Formel (XI), zunächst vorkondensiert. Hierzu setzt man eine wasserhaltige oder wasserfreie Metallverbindungen der Formel (VII) (von Anspruch 12) in einem vorzugsweise polaren Lösungsmittel oder Lösungsmittelgemisch mit einem Phosphin der allgemeinen Formel (VIII) bei einem molaren Verhältnis zwischen der Molzahl an Phosphineinheiten (VIII) und der Molzahl der insgesamt komplexgebundenen Metallatome von 1 : 1 bis 1000 : 1, vorzugsweise 1 : 1 bis 100 : 1, über einen Zeitraum von 1 Min. bis zu 48 Stunden um, setzt gegebenenfalls einen Teil oder die vollständige Menge einer oder mehrerer der Verbindungen der allgemeinen Formel (XI) der Lösung des gebildeten monomeren Metallkomplexes zu, kondensiert diese Mischung in Gegenwart einer nicht zur vollständigen Hydrolyse ausreichenden Menge Wasser, vorzugsweise von 1 bis 100 Mol% der hierzu benötigten Menge, über einen Zeitraum von 5 Min. bis zu 48 Stunden bei Raumtemperatur bis 200° C vor, setzt anschließend ein Aminosilan der Formel (X), gegebenenfalls die restliche oder vollständige Menge einer oder mehrerer der Verbindungen nach Formel (XI), gegebenenfalls weiteres Lösungsmittel und in jedem Fall weiteres Wasser zu, hydrolysiert erneut über einen Zeitraum bis zu 4 Stunden bevorzugt bei Rückflußtemperatur der Reaktionsmischung und verfährt anschließend hinsichtlich Gelierung und Weiterbehandlung des sich dabei bildenden Kondensats weiter wie vorstehend (nach Anspruch 12) beschrieben.

Die Vorkondensation kann generell durch den Zusatz einer geringen Menge eines sauren oder basischen oder eines Metall-haltigen Kondensationskatalysators beschleunigt werden.

Geeignete Katalysatoren sind anorganische oder organische Säuren oder Basen oder auch Zinnverbindungen. Die Menge Wasser, die zur Vorkondensation verwendet wird, hängt davon ab, welcher Oligomerisierungsgrad, d. h. welche Blockgröße erreicht werden soll. Bei Einsatz von mehr Wasser zur Vorkondensation entstehen natürlich größere Einheiten als bei Verwendung von weniger Wasser. Mit zu berücksichtigen ist bei der Wahl der zur Vorkondensation verwendeten Wassermenge natürlich eine etwa durch eine kristallwasserhaltige Metall-Ausgangskomponente nach Formel (VII) eingebrachte Wassermenge. Nach einer Variante des erfindungsgemäßen Verfahrens wird auf den Zusatz von freiem Wasser bei der Vorkondensation verzichtet und diese nur mit dem durch die kristallwasserhaltige Metallkomponente (VII) eingebrachten Wasser durchgeführt.

Nach einer weiteren Verfahrensvariante wird die zur Vorkondensation verwendete, über die gegebenenfalls vorhandene Kristallwassermenge hinausgehende Menge Wasser gleich zu Beginn der Umsetzung der Metallkomponente (VII) mit dem Phosphin (VIII) zugesetzt, so daß die Ausbildung des Monomerkomplexes und seine Vorkondensation, die Vorkondensation der überschüssigen Liganden sowie die der gegebenenfalls zugesetzten Verbindung(en) nach Formel (XI) gleichzeitig erfolgen. Direkt anschließend wird die vollständige Hydrolyse und Kondensation durchgeführt.

Die Dauer der Vorkondensation hängt, wie bereits vorstehend beschrieben, generell von der Hydrolysebereitschaft der monomeren Komponenten und der Temperatur ab.

Ein zweites erfindungsgemäßes Verfahren sieht vor, daß man eine oder mehrere wasserhaltige oder wasserfreie Metallverbindungen der Formel (VII) in einem vorzugsweise polaren Lösungsmittel mit einem Phosphin der allgemeinen Formel (VIII) in einem Verhältnis zwischen der Molzahl an Phosphineinheiten (VIII) und der Molzahl der insgesamt komplexgebundenen Metallatome von 1 : 1 bis x : 1, wobei x die jeweils metallspezifische maximale Koordinationszahl im jeweiligen Metallkomplex darstellt, über einen Zeitraum von 1 Min. bis zu 48 Stunden umsetzt, gegebenenfalls einen Teil oder die vollständige Menge einer oder mehrerer der Verbindungen der Formel (XI) zu der Lösung des gebildeten monomeren Metallkomplexes zugibt und diese Mischung in Gegenwart einer nicht zur vollständige Hydrolyse ausreichenden Menge Wasser, vorzugsweise von 1 bis 100 Mol% der hierzu benötigten Menge, über einen Zeitraum von 5 Min. bis zu 48 Stunden bei Raumtemperatur bis 200° C vorkondensiert, anschließend die über die maximale Koordinationszahl des Metalls hinausgehende Menge an Phosphin der Formel (VIII), gegebenenfalls die restliche oder vollständige Menge einer oder mehrerer der Verbindungen nach Formel (XI) sowie ein Aminosilan der Formel (X), gegebenenfalls weiteres Lösungsmittel und in jedem Fall weiteres Wasser zugibt, erneut über einen Zeitraum bis zu 4 Stunden, bevorzugt bei Rückflußtemperatur der Reaktionsmischung, hydrolysiert und anschließend, wie vorstehend, d. h. schon im Zusammenhang mit Anspruch 12 beschrieben, weiter verfährt.

Natürlich kann auch bei dieser und bei allen nachfolgenden Vorkondensationsvarianten ein saurer, basischer oder Metall-haltiger Kondensationskatalysator zugesetzt oder die Vorkondensation nur mit dem Kristallwasser einer wasserhaltigen Metall-Ausgangsverbindung oder die Vorkondensation zeitlich parallel zu der Umsetzung der Metallkomponente (VII) mit dem Phosphin (VIII) durchgeführt werden.

Ein drittes erfindungsgemäßes Verfahren, nach der sog. Block-Copolykondensate erhalten werden, bei denen eine Bildung von Blöcken gleicher Einheiten nach Formel (I) und (II) und gegebenenfalls einer oder mehrerer Einheiten nach Formel (IV) vorliegt, sieht vor, daß man den aus der Umsetzung der Metallverbindung nach Formel (VII) mit der Phosphinkomponente nach Formel (VIII) (gemäß Ansprüchen 12 bzw. 22) erhaltenen monomeren Metallkomplex zusammen mit gegebenenfalls vorhandenem überschüssigen Phosphin (VIII) bei oder nach seiner Herstellung vorkondensiert und ein Aminosilan der Formel (X) sowie gegebenenfalls eine oder mehrere Verbindungen der Formel (XI) jeweils unabhängig voneinander, ohne oder unter Verwendung eines Lösungsmittels, in Gegenwart einer nicht zur vollständigen Hydrolyse ausreichenden Menge Wasser, vorzugsweise in Gegenwart von 1 bis 100 Mol% der hierzu benötigten Menge, über einen Zeitraum von 5 Min. bis zu 48 Stunden bei Raumtemperatur bis 200° C vorkondensiert, anschließend die einzeln vorkondensierten Komponenten vereinigt und dann nach Zusatz von so viel Wasser, daß mindestens die für eine vollständige Hydrolyse stöchiometrisch erforderliche Menge Wasser anwesend ist, und gegebenenfalls weiterem Lösungsmittel, die vollständige Hydrolyse und Polykondensation sowie weitere Aufarbeitung (gemäß Anspruch 12) durchführt.

Ein viertes erfindungsgemäßes Verfahren, das vor allem ein deutlich unterschiedliches Gelierverhalten des gebildeten Phosphingruppen-haltigen Metallkomplexes und gegebenenfalls vorhandenem überschüssigen Phosphins (VIII) einerseits und Aminosilan (X) sowie einer oder mehrerer Verbindungen (XI) andererseits kompensieren soll, sieht vor, daß man die Metallverbindung (VII) mit dem Phosphin gemäß Ansprüchen 12 bzw. 22 umsetzt, gleichzeitig oder anschließend in Gegenwart einer nicht zur vollständigen Hydrolyse ausreichenden Menge Wasser, vorzugsweise in Gegenwart von 1 bis 100 Mol% der hierzu benötigten Menge, über einen Zeitraum von 5 Min. bis zu 48 Stunden bei Raumtemperatur bis 200° C vorkondensiert und, davon unabhängig, das Aminosilan (X), gegebenenfalls als Mischung mit einer oder mehreren Verbindungen der Formel (XI) ohne oder unter Verwendung eines Lösungsmittels, in Gegenwart einer nicht zur vollständigen Hydrolyse ausreichenden Menge Wasser, vorzugsweise in Gegenwart von 1 bis 100 Mol% der hierzu benötigten Menge, über einen Zeitraum von 5 Min. bis zu 48 Stunden bei Raumtemperatur bis 200° C vorkondensiert, anschließend die beiden Vorkondensate vereinigt und dann nach Zusatz weiteren Wassers und gegebenenfalls weiteren Lösungsmittels, so daß mindestens die für eine vollständige Hydrolyse stöchiometrisch erforderliche Menge Wasser anwesend ist, die vollständige Hydrolyse und Polykondensation sowie eine weitere Aufarbeitung gemäß Anspruch 12 durchführt.

Eine weitere erfindungsgemäße Verfahrensvariante sieht vor, daß man eine wasserfreie Metallkomponente (VII) mit der Phosphinkomponente (VIII) in bereits beschriebener Weise umsetzt aber nicht vorkondensiert und gleichzeitig, jeweils unabhängig voneinander, ein Aminosilan (X) sowie gegebenenfalls eine oder mehrere Verbindungen (XI) ohne oder unter Verwendung eines Lösungsmittels, in Gegenwart einer nicht zur vollständigen Hydrolyse ausreichenden Menge Wasser, vorzugsweise in Gegenwart von 1 bis 100 Mol% der hierzu benötigten Menge, über einen Zeitraum von 5 Min. bis zu 48 Stunden bei Raumtemperatur bis 200° C vorkondensiert, die nicht vorkondensierte metallhaltige Mischung und die beiden Vorkondensate miteinander vereinigt und dann nach Zusatz weiteren Wassers und gegebenenfalls weiteren Lösungsmittels, so daß mindestens die für eine vollständige Hydrolyse und Polykondensation stöchiometrisch erforderliche Menge Wasser anwesend ist, die vollständige Hydrolyse und Polykondensation sowie weitere Aufarbeitung gemäß Anspruch 12 durchführt.

Durch die unterschiedlichen Arten der Vorkondensation werden die Strukturen der später erhaltenen Polymeren entscheidend mitbestimmt. Diese wiederum beeinflussen die katalytischen Eigenschaften der so erhaltenen Katalysatoren und daneben u. a. auch die Haftung des Metalls bzw. der Metalle am Polymerligandträger.

Dies gilt auch für ein fünftes erfindungsgemäßes Verfahren, nach dem man eine wasserhaltige oder wasserfreie Metallverbindung (VII) in vorzugsweise polarem Lösungsmittel mit einem Phosphin (VIII) in Gegenwart eines Aminosilans (X) sowie gegebenenfalls einer oder mehrerer der Verbindungen (XI) über einen Zeitraum von 1 Min. bis zu 48 Stunden gemäß Ansprüchen 12 bzw. 22 umsetzt, der Lösung unter Rühren eine zumindest für die vollständige Hydrolyse und Kondensation ausreichende Menge Wasser zusetzt und dann weiter wie nach Anspruch 12 verfährt.

Natürlich kann auch bei dieser Verfahrensweise, z. B. um ein unterschiedliches Gelierverhalten der Komponenten auszugleichen, eine gezielte Vorkondensation in der Weise durchgeführt werden, daß während der Umsetzung der Komponenten zum monomeren Metallkomplex (gemäß Anspruch 26) oder daran anschließend durch Zusatz einer nicht zur vollständigen Hydrolyse ausreichenden Menge Wasser, vorzugsweise von 1 bis 100 Mol% der hierzu benötigten Menge, über einen Zeitraum von 5 Min. bis zu 48 Stunden bei Raumtemperatur bis 200° C, (d. h. nach Anspruch 18) vorkondensiert wird und dann nach Zusatz weiteren Wassers und gegebenenfalls weiteren Lösungsmittels, so daß mindestens die für eine vollständige Hydrolyse und Polykondensation stöchiometrisch erforderliche Menge Wasser anwesend ist, die vollständige Hydrolyse und Polykondensation gemäß Anspruch 12 durchgeführt wird.

Eine spezielle Verfahrensvariante, die zur Herstellung von polymeren geformten heterogenisierten Komplexkatalysatoren führt, bei denen gemäß Formel (VI) X = H ist oder das Metall komplexgebunden in nullwertiger Form vorliegt, sieht eine Behandlung des nach den Verfahrensweisen gemäß Ansprüchen 12 bis 27 primär hergestellten monomeren Metallkomplexes vor oder nach einer gegebenenfalls durchgeführten Vorkondensation mit einem Reduktionsmittel, gegebenenfalls bei erhöhter Temperatur und/oder Überdruck über einen Zeitraum von 1 Min. bis zu 48 Stunden vor und schließt wie nach Anspruch 12 die weitere Hydrolyse, Polykondensation und Aufarbeitung an.

Geeignete Reduktionsmittel sind z. B. Formaldehyd, Hydrazin, Alkali- oder Erdalkalimetallborhydrid, Boranverbindungen, Formiate, Aluminiumhydride bzw. auch nur Alkohole oder Wasserstoff. Zusätzlich zu dem Reduktionsmittel kann darüber hinaus auch noch ein separater Säureakzeptor neben dem bereits vorhandenen Amin (X) oder überschüssigen Phosphin (VIII) der Metallkomplex-haltigen Lösung zugesetzt werden. Geeignet sind z. B. Alkali- oder Erdalkalimetallhydroxide, Alkalimetall- oder Erdalkalimetallhydride, komplexe Bor-oder Aluminiumhydride, Alkali- oder Erdalkalimetallcarbonate oder -bicarbonate, primäre, sekundäre oder tertiäre Amine.

Nach einer Abwandlung der vorstehend angegebenen Verfahrensvariante wird der gemäß Ansprüchen 12 bis 27 primär hergestellte monomere Metallkomplex zunächst hydrolysiert und unter Formung polykondensiert und vor oder nach mindestens einer der gemäß Anspruch 12 vorgesehenen Aufarbeitungsstufen, suspendiert in Wasser oder einem Lösungsmittel, bevorzugt einem niederen Alkohol oder einem Gemisch desselben mit Wasser, noch einer Reduktionsbehandlung nach Anspruch 28, gegebenenfalls unter Überdruck, unterworfen. Es wird also die reduktive Behandlung nach der Ausbildung des geformten Komplexkatalysators (d. h. nach dem Zusatz des Dispergierwassers gemäß Anspruch 12) oder auch nach der Extraktion des gebildeten geformten Metallkomplexes oder auch nach dessen Trocknung und gegebenenfalls Temperung durchgeführt, und zwar in Suspension mit einem geeigneten Lösungsmittel als Suspensionsmittel. Bevorzugt wird dafür Wasser oder ein niederer Alkohol oder ein Gemisch eines solchen mit Wasser verwendet.

Eine besonders wichtige Ausführungsform aller erfindungsgemäßen Verfahren sieht vor, den noch lösungsmittel- und wasserfeuchten bzw. -nassen kugelförmigen Komplex einer Temperaturbehandlung zu unterwerfen.

Diese Behandlung unter "dämpfenden" bzw. digerierenden Bedingungen dient ebenfalls überwiegend einer Verbesserung der mechanischen Festigkeit und der Porosität des geformten Materials und kann auch in der zuletzt vorliegenden, eine flüssige und die feste Produktphase enthaltenden Dispersion des Herstellungsganges oder in Wasser allein durchgeführt werden. Die Temperaturbehandlung kann auch mit einer reduktiven Behandlung kombiniert werden.

Die vorstehend beschriebene Ausführungsform einer Nachbehandlung der erhaltenen, aber nicht getrockneten geformten Komplexkatalysatoren besteht somit darin, den in Form von Kugeln ausgebildeten Komplex in Anwesenheit mindestens der Komponente Wasser bzw. der im Herstellungsgang zuletzt vorliegenden flüssigen Phase als Dampf oder Flüssigkeit, einer Temperaturbehandlung während 1 Stunde bis zu 1 Woche bei Temperaturen von 50 - 300° C, vorzugsweise 100 - 200° C, gegebenenfalls unter Überdruck, zu unterwerfen. Dabei kann die Anwesenheit eines sauren, basischen oder zusätzlichen, Metall-haltigen Katalysators von Vorteil sein. Diese Nachbehandlung kann in Verbindung mit einer reduktiven Behandlung durchgeführt werden. Eine bevorzugte Methode ist die Wasserstoffbehandlung; dazu können auch Gemische zwischen Wasserstoff und Inertgasen verwendet werden. Eine besonders wirksame Reduktion kann durch Einsatz von Natriumborhydrid erfolgen; eine Kombination dieses Agens mit $H_2$ ist ebenfalls möglich.

Charakterisiert sind die neuen geformten polymeren Übergangsmetall-Komplexkatalysatoren insbesondere anhand der quantitativen Hydrolyseausbeuten, der Elementaranalysen und durch das katalytische Verhalten, das komplexspezifisch jeweils vergleichbar ist mit dem eines analogen homogenen Komplexkatalysators.

Zwischen den nach den unterschiedlichen Herstellungsverfahren erhaltenen polymeren Katalysatoren besteht rein optisch kein Unterschied. Ein wichtiges Charakteristikum der nach den erfindungsgemäßen Verfahren hergestellten Katalysatoren ist die Tatsache, daß das komplexgebundene Metall homogen dispers, d. h. gleichmäßig über das geformte Partikel verteilt ist. Um den Zugang der umzusetzenden Edukte zu den innenliegenden Katalysezentren zu ermöglichen, ist es notwendig, daß die geformten Katalysatoren geeignete physikalische Eigenschaften aufweisen. Neben einem geeigneten Partikeldurchmesser von 0,01 bis 3,0 mm, vorzugsweise 0,05 bis 2,0 mm, zählt hierzu eine

spezifische Oberfläche von > 0 bis 1000 $m^2/g$, vorzugsweise > 0 bis 700 $m^2/g$, ein spezifisches Porenvolumen von 0,01 bis 6,5 ml/g sowie eine Schüttdichte von 50 - 1000 g/l, vorzugsweise 100 bis 800 g/l. Die einstellbaren Porendurchmesser liegen im Bereich von > 0 bis 1000 nm. Die thermische Stabilität der geformten Katalysatoren beträgt in Abhängigkeit vom gebildeten Komplextyp an der Luft mehr als 130° C und unter Inertgasatmosphäre mehr als 200° C.

Die erfindungsgemäßen geformten Übergangsmetall-Komplexkatalysatoren stellen wertvolle Katalysatoren für chemische Umsetzungen wie Hydroformylierungs-, Hydrierungs-, Oligomerisierungs-, Carbonylierungs-, Hydrosilylierungs-, Carboximethylierungs- und Isomerisierungsreaktionen sowie für Reaktionen von CO oder $CO_2$ mit $H_2$ dar. Eine entsprechende Verwendung stellt daher einen weiteren Gegenstand der Erfindung dar.

Metallspezifisch zeigt sich dabei genau analog wie bei homogenen Katalysatoren eine unterschiedliche Eignung der erfindungsgemäßen Systeme für die o.g. Reaktionen. Die geformten polymeren Metallkomplex-Katalysatoren können in Suspension oder im Festbett oder im Fließbett für Reaktionen in flüssiger oder gasförmiger Phase eingesetzt werden.

Die Erfindung wird nachfolgend anhand von Ausführungsbeispielen weiter erläutert.

Beispiel 1 (Statistisches Copolykondensat)

14,54 g (0,03 Mol) $[RhCl(C_8H_{12})]_2$ ($C_8H_{12}$ = Cyclooctadien und 62,7 g (0,18 Mol) $(C_6H_5)_2P(CH_2)_3Si(OCH_3)_3$ wurden in 100 ml Ethanol vereinigt. Die Mischung wurde in einem 4 l-Glasgefäß mit Rührer und Rückflußfühler auf Rückflußtemperatur aufgeheizt und 1 Stunde bei dieser Temperatur gerührt. Anschließend wurden 223,1 g (0,35 Mol) $N[(CH_2)_3Si(OC_2H_5)_3]_3$, 250 ml Ethanol und 73,8 g (0,35 Mol) $Si(OC_2H_5)_4$ zu der Mischung zugegeben. Die klare Lösung wurde wieder auf Rückflußtemperatur aufgeheizt und dann mit 100 ml entsalztem Wasser versetzt.

Es wurde noch 10 Min. unter Rückfluß gerührt, dann auf 75° C abgekühlt und solange weitergerührt, bis die Gelierung einsetzte. 2 Min. nach dem Einsetzen der Gelierung wurden zu der Mischung 750 ml Octanol-1 und nach weiteren 5 Min. 700 ml entsalztes Wasser zugesetzt. Das 2-Phasengemisch wurde unter Rühren (500 U/min.) wieder auf Rückflußtemperatur aufgeheizt, 2 Stunden bei dieser Temperatur gerührt, dann abgekühlt und in einen 4 1-Druckbehälter überführt. Die Suspension wurde 24 Stunden bei 130° C und einem Eigendruck von ca. 8 bar langsam gerührt, dann wiederum abgekühlt und die flüssige Phase von dem in Form von Kugeln vorliegenden Feststoff abgesaugt. Nach 2-maliger Extraktion mit je 2 1 Ethanol wurde das Produkt in einen Trockenschrank überführt und zunächst 8 Stunden bei 80°C und dann 16 Stunden bei 130° C unter $N_2$-Atmosphäre getrocknet. Erhalten wurden 92,6 g (ca. 99,1 % d. Theorie) eines geformten polymeren Rhodium-Komplexkatalysators, bestehend aus Polymereinheiten der Formel

$$RhCl\{(C_6H_5)_2P\text{-}(CH_2)_3SiO_{3/2} \cdot 2N[(CH_2)_3SiO_{3/2}]_3 \cdot 2SiO_2\}_3$$

von dem 98 % eine Korngröße von 0,3 bis 1,8 mm besaßen.

| | |
|---|---|
| Spezifische Oberfläche | 612 $m^2/g$ |
| Spezifisches Gesamtporenvolumen | 2,2 ml/g |
| Schüttdichte | 395 g/l |

| Elementaranalysen | % Rh | % Cl | % P | % Si |
|---|---|---|---|---|
| Theorie | 3,3 | 1,1 | 3,0 | 24,3 |
| Gefunden | 3,4 | 1,1 | 2,9 | 23,8 |

Beispiel 2 (Gemischtes Copolykondensat)

1,66 g (0,005 Mol) $RhCl_3(CH_3CN)_3$ und 9,1 g (0,1 Mol) $(C_6H_5)P\text{-}(CH_2)_3Si(OC_2H_5)_3$ wurden in 100 ml Ethanol vereinigt. Die Mischung wurde auf Rückflußtemperatur aufgeheizt und mit 5 ml entsalztem Wasser versetzt. Die Lösung wurde eine Stunde bei dieser Temperatur gerührt, dann mit 63,0 g (0,1 Mol) $N[(CH_2)_3Si(OC_2H_5)_3]_3$ sowie mit weiteren 20 ml Wasser versetzt und noch 25 Min. unter Rückfluß gerührt. Es wurde auf 70° C abgekühlt und solange bei dieser Temperatur mit 50 U/min. gerührt, bis die Gelierung einsetzte. Sofort nach dem Einsetzen der Gelierung wurden dem sich bildenden Gel 160 ml Xylol (techn. Gemisch) und nach einer weiteren Minute 300 ml Wasser zugesetzt. Das 2-Phasensystem wurde 1 Stunde unter Rückfluß gerührt, dann abgekühlt und in einen 3 1-Druckbehälter überführt. Die Suspension wurde 48 Stunden bei 140° C gehalten und dann analog zu Beispiel 1 getrocknet sowie weitere 12 Stunden bei 160° C getempert. Erhalten wurden 56,8 g (96,9 % d. Theorie) eines geformten polymeren Rhodium-Komplexkatalysators, bestehend aus Polymereinheiten der Formel

$$RhCl_3\{(C_6H_5)_2P\text{-}(CH_2)_3SiO_{3/2} \cdot N[(CH_2)_3SiO_{3/2}]_3\}_{20},$$

von dem 98 % eine Korngröße von 0,3 bis 1,8 mm besaßen.

| | |
|---|---|
| Spezifische Oberfläche | 690 $m^2/g$ |
| Spezifisches Gesamtporenvolumen | 1,5 ml/g |
| Schüttdichte | 400 g/l |

| Elementaranalysen | % Rh | % Cl | % P |
|---|---|---|---|
| Theorie | 0,9 | 0,9 | 5,3 |
| Gefunden | 0,9 | 0,8 | 5,1 |

<u>Beispiel 3</u> (Block-Copolykondensat)

0,88 g (0,002 Mol) $[Rh(O_2CCH_3)_2]_2$, 40,5 g (0,1 Mol) $(C_6H_5)_2P\text{-}CH_2Si(OC_3H_7)_3$ und 7,4 g (0,05 Mol) $(CH_3)_2Si(OC_2H_5)_2$ wurden in 70 ml Isopropanol vereinigt. Die Lösung wurde mit 8 ml entsalztem Wasser versetzt, auf Rückflußtemperatur aufgeheizt und 3 Stunden unter Rückfluß gerührt. Parallel dazu wurden 24,1 g (0,05 Mol) $HN[(CH_2)_8Si(OCH_3)_3]_2$ und 5 ml 1 %ige wäßrige $NH_3$-Lösung in 50 ml Isopropanol vereinigt und ebenfalls 2 Stunden unter Rückfluß gerührt. Anschließend wurden die beiden Vorkondensate vereinigt, 15 ml Wasser zugegeben und die Mischung weiter unter Rückfluß gerührt bis die Gelierung einsetzte. 10 Min. nach dem Einsetzen der Gelierung wurden 200 ml sec.-Butanol und nach weiteren 30 Min. 150 ml entsalztes Wasser zugesetzt. Das 2-Phasensystem wurde insgesamt 10 Stunden unter Rückfluß gerührt, dann abgekühlt und der Feststoff von der flüssigen Phase abgetrennt. Nach Trocknung analog zu Beispiel 2 wurden 46,2 g (98,5 % d. Theorie) eines polymeren Komplexkatalysators, bestehend aus Polymereinheiten der Formel

$$Rh(O_2CCH_3)_2\{(C_6H_5)_2P\text{-}CH_2\text{-}SiO_{3/2} \cdot 0{,}5HN[(CH_2)_8SiO_{3/2}]_2 \cdot 0{,}5(CH_3)_2SiO_{2/2}\}_{25}$$

mit einer Korngrößenverteilung von 0,1 mm bis 1,8 mm erhalten.

| | |
|---|---|
| Spezifische Oberfläche | 131 $m^2/g$ |
| Spezifisches Gesamtporenvolumen | 0,5 ml/g |
| Schüttdichte | 490 g/l |

| Elementaranalysen | % Rh | % P | % Si |
|---|---|---|---|
| Theorie | 0,9 | 6,6 | 15,0 |
| Gefunden | 0,9 | 6,3 | 14,7 |

<u>Beispiel 4</u>

15,7 g (0,09 Mol) $PdCl_2$, 62,7 g (0,18 Mol) $(C_6H_5)_2P\text{-}(CH_2)_3Si(OCH_3)_3$ und 73,7 g (0.35 Mol) $Si(OC_2H_5)_4$ wurden in 300 ml Methanol vereinigt. Die Mischung wurde auf Rückflußtemperatur aufgeheizt und zunächst solange unter Rückfluß gerührt, bis alles $PdCl_2$ gelöst war. Dann wurden zu der Lösung 10 ml Wasser zugegeben und unter Rühren bei Rückflußtemperatur zunächst 2 Stunden vorkondensiert. Anschließend wurden 178,3 g (0,35 Mol) $N[(CH_2)_3Si(OCH_3)_3]_3$ sowie weitere 100 ml Wasser zugesetzt und noch 25 Min. unter Rückfluß gerührt. Dann wurde die Lösung auf 60° C abgekühlt, bei dieser Temperatur weiter gerührt, bis die Gelierung einsetzte. Sofort nach dem Einsetzen der Gelierung wurden dem sich bildenden Gel 500 ml 2-Ethylhexanol und nach weiteren 3 Min. 500 ml Wasser zugesetzt. Das 2-Phasensystem wurde wieder auf Rückflußtemperatur aufgeheizt und 2 Stunden bei dieser Temperatur gerührt. Nach weiterer Verfahrensweise analog zu Beispiel 1, mit dem Unterschied einer 48-stündigen Nachbehandlung bei 140° C, wurden 192,9 g (99,1 % d. Theorie) eines geformten polymeren Palladium-Komplexkatalysators, bestehend

aus Polymereinheiten der Formel

$$PdCl_2\{(C_6H_5)_2P\text{-}(CH_2)_3\text{-}SiO_{3/2}\cdot 2N[(CH_2)_3SiO_{3/2}]_3\cdot 2SiO_2\}_2$$

erhalten. 95 % der gebildeten Kugeln besaßen einen Durchmesser von 0,05 bis 1,0 mm.

| | |
|---|---|
| Spezifische Oberfläche | 738 $m^2/g$ |
| Spezifisches Gesamtporenvolumen | 4,6 ml/g |
| Mesoporenvolumen | 2,4 ml |
| Makroporenvolumen | 2,2 ml |
| Schüttdichte | 230 g/l |

| Elementaranalysen | % Pd | % P | % N |
|---|---|---|---|
| Theorie | 4,9 | 2,9 | 2,6 |
| Gefunden | 4,8 | 2,9 | 2,4 |

Beispiel 5

2,94 g (0,01 Mol) $Na_2PdCl_4$, 15,6 g (0,04 Mol) $(C_6H_5)_2P\text{-}(CH_2)_3\text{-}Si(OC_2H_5)_3$, 17,03 g (0,04 Mol) $HN[(CH_2)_3Si(OC_2H_5)_3]_2$ und 16,51 g (0,08 Mol) $C_3H_7Si(OC_2H_5)_3$ wurden in 60 ml Ethanol vereinigt. Die Mischung wurde in einem 0,5 l-Glasgefäß auf Rückflußtemperatur aufgeheizt und 20 Min. bei dieser Temperatur gerührt. Es wurden 30 ml Hexanol-1 und 15 ml Wasser zugesetzt, dann die Lösung auf 40° C abgekühlt und solange weiter gerührt, bis die Gelierung einsetzte. Sofort nach dem Einsetzen der Gelierung wurden weitere 80 ml Hexanol und nach einer halben Minute des Homogenisierens 120 ml Wasser zugegeben. Das 2-Phasensystem wurde auf Rückflußtemperatur aufgeheizt und 3 Stunden bei dieser Temperatur gerührt. Dann wurde abgekühlt und der gebildete Polymerkomplex von der flüssigen Phase abfiltriert und 2 mal mit je 300 ml Ethanol gewaschen. Nach 8-stündiger Trocknung bei 100° C und 16-stündiger Trocknung bei 140° C unter $N_2$-Atmosphäre wurden 28,6 g (97,7 % d. Theorie) eines polymeren Komplexes, bestehend aus Einheiten der Formel

$$PdCl_2\{(C_6H_5)_2P\text{-}(CH_2)_3SiO_{3/2}\cdot HN[(CH_2)_3SiO_{3/2}]_{23}H_7SiO_{3/2}\}_4$$

erhalten. 98 % der gebildeten Kugeln besaßen einen Durchmesser von 0,2 bis 1,6 mm.

| | |
|---|---|
| Spezifische Oberfläche | 326 $m^2/g$ |
| Schüttdichte | 405 g/l |

| Elementaranalysen: | % Pd | % P | % N |
|---|---|---|---|
| Theorie: | 3,7 | 4,3 | 2,0 |
| Gefunden: | 3,8 | 4,2 | 2,1 |

Beispiel 6 (Vorkondensation ohne Zusatz von Wasser nur mit Kristallwasser)

22.26 g (63,2 mMol), $IrCl_3\cdot 3H_2O$ wurden in einem 3 1-G1asgefäß mit Doppelmantel-Beheizung, KPG-Rührer und Rückflußkühler in 500 ml Ethanol unter Argonatmosphäre bei 60° C gelöst. Die klare Lösung wurde zunächst mit 66,0 g (189,5 mMol) $(C_6H_5)_2P\text{-}(CH_2)_3Si(OCH_3)_3$ und nach 5 Min. mit 39,5 g (189,5 mMol) $Si(OC_2H_5)_4$ versetzt und anschließend über einen Zeitraum von 1 Stunde bei Rückflußtemperatur gerührt, wobei gleichzeitig Umsetzung und Vorkondensation stattfanden. Anschließend wurden nochmals 39,5 g $Si(OC_2H_5)_4$, 238,8 g (379,0 mMol) $N[(CH_2)_3Si(OC_2H_5)_3]_3$ und 130 ml $H_2O$ zugesetzt. Nach 10-minütigem weiteren Rühren unter Rückflußtemperatur wurde die Lösung auf 70° C abgekühlt und solange weiter bei dieser Temperatur mit 100 U/Min. gerührt, bis die Gelierung einsetzte.

Sofort nach dem Einsetzen der Gelierung wurden 700 ml 60° C warmes Octanol-1 dem sich bildenden Gel zugesetzt und die Rührgeschwindigkeit auf 750 U/Min. erhöht. Nach einer weiteren Minute des Homogenisierens wurden

zu der viskosen Lösung 1200 ml Wasser, in dem 1,2 g Polyvinylalkohol (Mowiol®) gelöst worden waren, zugegeben. Das 2-Phasensystem wurde auf Rückflußtemperatur aufgeheizt und weitere 2 Stunden bei dieser Temperatur gerührt. Nach dem Abkühlen wurde der in Form von kleinen gelben Kügelchen vorliegende Feststoff und die Mutterlösung durch Dekantieren separiert und lösungsmittelfeuchter Feststoff und Mutterlösung in 2 gleiche Teile geteilt. Eine Hälfte des genannten Festoffes sowie die halbe Menge Mutterlösung wurde in einen 5 1-Autoklaven überführt (Weiterverarbeitung der anderen Produkthälfte siehe Beispiel 7) und unter Rühren bei einer Temperatur von 135° C über einen Zeitraum von 48 Stunden unter Eigendruck gerührt. Es wurde abgekühlt, die flüssige Phase vom Feststoff abgesaugt und dieser 2 mal mit je 1 l Ethanol gewaschen. Anschließend wurde 12 Stunden bei 100° C und 12 Stunden bei 130° C unter $N_2$-Atmosphäre getrocknet. Erhalten wurden 103,0 g (99,5 % d. Theorie) Produkt, von dem über 98 % in Form von gelben Kugeln mit einem Kugeldurchmesser von 50 µm bis 0,6 mm vorlagen.

| Elementaranalysen | % Ir | %P | % H | % C | % Cl | % Si |
|---|---|---|---|---|---|---|
| Theorie | 5,9 | 2,8 | 4,8 | 36,3 | 3.2 | 23,1 |
| Gefunden | 5,8 | 2,8 | 4,7 | 35,8 | 3,3 | 22,8 |

| | |
|---|---|
| Schüttdichte | 250 g/l |
| Spezifische Oberfläche | 648 $m^2/g$ |
| Porenvolumen (Porendurchmesser größer 2 nm) | 3,4 ml/g |

Formel für Polymereinheit:

$$IrCl_3\{(C_6H_5)_2P\text{-}(CH_2)_3SiO_{3/2}\cdot 2N[(CH_2)_3SiO_{3/2}]_3\cdot 2SiO_2\}_3$$

Beispiel 7

Die zweite Hälfte des in Beispiel 6 hergestellten polymeren Produkts wurde einer reduktiven Behandlung mit Natriumborhydrid unterzogen. Hierzu wurde der geformte lösungsmittelfeuchte Feststoff zusammen mit der zweiten Hälfte der Mutterlösung in einen Autoklaven überführt und 35 g NaBH4 zugesetzt. Der sofort gebildete Wasserstoff wurde zunächst abgelassen und 2 mal mit Argon gespült. Dann wurde auf 140° C aufgeheizt, wobei sich ein Druck von 28 bar einstellte, und 24 Stunden bei dieser Temperatur gerührt. Nach dem Abkühlen, Absaugen der flüssigen Phase wurde 2 mal mit je 1 l Ethanol, 2 mal mit je 1 l Wasser. noch 2 mal mit je 1 l Ethanol gewaschen und dann der hellgelbe Feststoff 12 Stunden bei 100° C sowie 12 Stunden bei 130° C unter $N_2$-Atmosphäre getrocknet. Es wurden 99,8 g (99,5 % d. Theorie) Polymerkomplex, bestehend aus Polymereinheiten der Formel

$$IrH_3\{(C_6H_5)_2P\text{-}(CH_2)_3SiO_{3/2}\cdot 2N[(CH_2)_3SiO_{3/2}]_3\cdot 2SiO_2\}_3$$

erhalten. 98 % des erhaltenen Produkts lagen in Form von Kugeln mit einem Durchmesser von 50 µm bis 0,6 mm vor.

| | |
|---|---|
| Schüttdichte | 210 g/l |

| Elementaranalysen | % Ir | % P | % Cl |
|---|---|---|---|
| Theorie | 6,1 | 2,9 | 0,0 |
| Gefunden | 6,0 | 2,8 | 0,02 |

| | |
|---|---|
| Spezifische Oberfläche | 483 $m^2/g$ |

Beispiel 8

17.49 g (63,2 mMol) $RuCl_3\cdot 3H_2O$ wurden in 125 ml Ethanol bei 60° C gelöst, dann mit 66,1 g (189,6 mMol) $(C_6H_5)_2P\text{-}(CH_2)_3Si(OCH_3)_3$ und mit 5 ml Wasser vereinigt. Die Lösung wurde dann über einen Zeitraum von 2 Stunden

bei Rückflußtemperatur unter Rühren vorkondensiert. Parallel dazu wurden 164,8 g (379,0 mMol) $Si(OC_2H_5)_4$, gelöst in 50 ml Ethanol, durch Reaktion mit 5 ml Wasser und 238,8 g (379,0 mMol) $N[(CH_2)_3Si(OC_2H_5)_3]_3$, gelöst in 200 ml Ethanol, durch Reaktion mit 8 ml Wasser über einen Zeitraum von jeweils 2 Stunden bei Rückflußtemperatur unter Rühren vorkondensiert. Im Anschluß daran wurden alle 3 Vorkondensate in einem 3 l-Glasgefäß mit Doppelmantelbeheizung, KPG-Rührer sowie Rückflußkühler vereinigt, das Gemisch mit weiteren 50 ml Wasser versetzt und nochmals 10 Min. unter Rückfluß gerührt. Danach wird auf 70° C abgekühlt und solange weiter gerührt, bis die Gelierung einsetzte. 5 Min. nach dem Einsetzen der Gelierung wurden dem sich bildenden Gel 750 ml Octanol und nach weiteren 2 Min. 1300 ml Wasser zugesetzt. Das 2-Phasensystem wurde wieder auf Rückflußtemperatur aufgeheizt und 1 Stunde bei dieser Temperatur gerührt. Anschließend wurde der Ansatz abgekühlt und der gebildete Feststoff sowie die Mutterlösung in jeweils 2 gleiche Teile geteilt. Jeweils ein Teil davon wurde in einen 5 l-Autoklaven überführt und hier 24 Stunden bei 150° C gerührt. Nach Abkühlung, Absaugen der flüssigen Phase, 3-maliger Extraktion des gelben Feststoffs mit je 500 ml Ethanol und 8-stündiger Trocknung bei 110° C sowie 12-stündiger Trocknung bei 140° C wurden 100,3 g (99,7 % d. Theorie) Polymerkomplex, bestehend aus Polymereinheiten der Formel

$$RuCl_3\{(C_6H_5)_2P\text{-}(CH_2)_3SiO_{3/2} \cdot 2N[(CH_2)_3SiO_{3/2}]_3 \cdot 2SiO_2\}_3$$

erhalten. 96 % des erhaltenen Produkts lagen in Form von Kugeln mit einem Durchmesser von 0,2 bis 1,2 mm vor.

| | |
|---|---|
| Schüttdichte | 320 g/l |
| Gesamtporenvolumen | 3,4 ml/g (Porendurchmesser: 2 bis 1000 nm) |

| Elementaranalysen | % Ru | % P | % H | % C | % Cl | % Si | % N |
|---|---|---|---|---|---|---|---|
| Theorie | 3,2 | 2,9 | 4,9 | 37,3 | 3,3 | 23,8 | 2,6 |
| Gefunden | 3,2 | 2,8 | 4,8 | 36,8 | 3,3 | 23,7 | 2,5 |

Beispiel 9

Die andere Hälfte des in Beispiel 8 hergestellten, kugelförmigen, noch lösungsmittelfeuchten Rohprodukts wurde zusammen mit der anderen Hälfte Menge an Mutterlösung in einen Autoklaven überführt und dann mit 20 g Natriumborhydrid versetzt. Nach einer Verfahrensweise analog zu Beispiel 7 wurden 97,0 g (99,6 % d. Theorie) Polymerkomplex, bestehend aus Einheiten der Formel

$$RuH_2\{(C_6H_5)_2P\text{-}(CH_2)_3SiO_{3/2} \cdot 2N[(CH_2)_3SiO_{3/2}]_3 \cdot 2SiO_2\}_3$$

erhalten.

| | |
|---|---|
| Schüttdichte: | 185 g/l |

| Elementaranalysen | % Ru | % P | % H | % C | % Si | % Cl | % N |
|---|---|---|---|---|---|---|---|
| Theorie | 3,3 | 3,0 | 5,1 | 38,6 | 24,6 | 0 | 2,7 |
| Gefunden | 3,2 | 2,9 | 5,0 | 38,2 | 23,3 | 0,02 | 2,8 |

Beispiel 10

36,1 g (95 mMol) $(NH_4)_2PtCl_4$, 132,4 g (380 mMol) $(C_6H_5)_2P\text{-}(CH_2)_3Si(OCH_3)_3$ und 158,3 g (760 mMol) $Si(OC_2H_5)_4$ wurden in einem 3 l-Autoklaven in 400 ml Ethanol vereinigt. Die Mischung wurde zunächst 1 Stunde bei 100° C gerührt, dann mit 15 g 35 %iger $N_2H_4$-Lösung sowie 6,6 g NaOH versetzt und und weitere 2 Stunden bei 120° C gerührt. Im Anschluß daran wurde die Lösung in ein Glasgefäß mit KPG-Rührer und Rückflußkühler überführt und mit 119,6 g (190 mMol) $N[(CH_2)_3Si(OC_2H_5)_3]_3$ und weiteren 120 ml Wasser versetzt und auf 65° C abgekühlt. Bei dieser Temperatur wurde solange weiter gerührt, bis die Gelierung einsetzte. Sofort nach dem Einsetzen der Gelierung wurden 750 ml Octanol und nach weiteren 6 Min. 800 ml Wasser zugegeben. Es wurde noch eine halbe Stunde bei Rückflußtemperatur

mit 500 U/Min. gerührt und dann die gesamte Suspension in einen Autoklaven überführt. Nach 24-stündiger Nachbehandlung bei 150° C wurde der Feststoff 2 mal mit je 1 l Ethanol und 2 mal mit je 1 l Wasser extrahiert und dann 24 Stunden bei 120° C sowie 100 mbar Druck getrocknet. Erhalten wurden 226,0 g (99,7 % d. Theorie) Polymerkomplex, bestehend aus Polymereinheiten der Formel

$$Pt\{(C_6H_5)_2P\text{-}(CH_2)_3SiO_{3/2}\cdot 0{,}5N[(CH_2)_3SiO_{3/2}]_3\cdot 2SiO_2\}_4$$

95 % des in Form von Kugeln vorliegenden Produkts besaßen einen Teilchendurchmesser von 0,1 - 1,8 mm.

| | |
|---|---|
| Schüttdichte | 230 g/l |

| Elementaranalysen | % Pt | % Cl | % P | % N | % Si |
|---|---|---|---|---|---|
| Theorie | 8,2 | 0 | 5,2 | 1,2 | 21,2 |
| Gefunden | 7,9 | 0.05 | 4,9 | 1,1 | 22,0 |

Beispiel 11

13,5 g (50 mMol) $FeCl_3\cdot 3H_2O$ und 67,9 g (150 mMol)

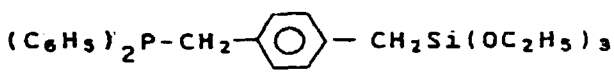

wurden in 500 ml Ethanol gelöst. Die Lösung wurde eine Stunde unter Rückfluß gerührt, dann mit 377,9 g (750 mMol) $N[(CH_2)_3Si(OCH_3)_3]_3$ und 140 ml $H_2O$ versetzt. Es wurde weiter unter Rückfluß gerührt bis die Gelierung einsetzte. Sofort nach dem Gelieren wurden 1000 ml 2-Ethylhexanol und nach einer weiteren Minute des Homogenisierens 10,6 g (50 mMol) $(H_5C_2)Ti(OC_2H_5)_3$ sowie 1000 ml Wasser zugegeben. Das 2-Phasensystem wurde noch 2 Stunden unter Rückfluß gerührt, dann abgekühlt, die flüssige Phase abgesaugt und der verbliebene Feststoff 3 mal mit je 1 l Ethanol extrahiert. Nach 8-stündiger Trocknung bei 100° C und 12-stündiger Trocknung bei 130° C sowie 12-stündiger Trocknung bei 160° C unter $N_2$-Atmosphäre wurden 285 g (99,4 % d. Theorie) geformtes Polymerprodukt, bestehend aus Einheiten der Formel

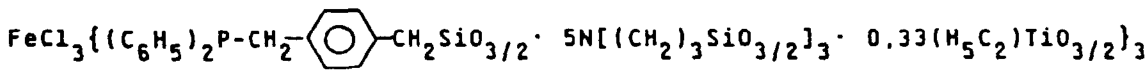

erhalten.

| | |
|---|---|
| Kugelgröße ($d_{95\,\%}$) | 0,3 - 2,0 mm |
| Schüttdichte | 410 g/l |

| Elementaranalysen | % Fe | % P | % N | % Ti |
|---|---|---|---|---|
| Theorie | 1,0 | 1,6 | 3,7 | 0,8 |
| Gefunden | 0,9 | 1,5 | 3,6 | 0,8 |

Beispiel 12

Ausgehend von 12,5 g (50 mMol) $Co(O_2CCH_3)_2\cdot 4H_2O$, 48,1 g (150 mMol) $(C_6H_5)_2P\text{-}CH_2\text{-}Si(OCH_3)_3$ und 377,g (750 mMol) $N[(CH_2)_3Si(OCH_3)_3]_3$ sowie 7,4 g (30 mMol) $Al(OC_4H_9)_3$ wurden unter Einsatz derselben Lösungsmittel sowie Lösungsmittelmengen und unter Praktizierung derselben Verfahrensweise wie in Beispiel 11 268 g Polymerkomplex, bestehend aus Polymereinheiten der Formel

$$Co(O_2CCH_3)_2\{(C_6H_5)_2P\text{-}CH_2\text{-}SiO_{3/2}\cdot 5N[(CH_2)_3SiO_{3/2}]_3\cdot 0{,}2AlO_{3/2}]_3$$

erhalten.

| | |
|---|---|
| Kugelgröße ($d_{98\,\%}$) | 0,2 - 2,0 mm |
| Schüttdichte | 360 g/l |

| Elementaranalysen: | % Co | % P | % N | % Al |
|---|---|---|---|---|
| Theorie: | 1,1 | 1,7 | 3,9 | 0,3 |
| Gefunden: | 1,0 | 1,8 | 3,9 | 0,2 |

Beispiel 13

Ausgehend von 13,1 g (50 mMol) $NiSO_4 \cdot 6H_2O$, 19,5 g (50 mMol) $(C_6H_5)_2P\text{-}(CH_2)_3Si(OC_2H_5)_3$ und 630,06 g (1,0 mMol) $N[(CH_2)_3Si(OC_2H_5)_3]_3$ sowie 19,2 g (50 mMol) $Zr(OC_4H_9)_4$ wurden unter Einsatz von Diisopropylether anstelle von 2-Ethylhexanol und unter Praktizierung derselben Verfahrensweise wie in Beispiel 11 321,0 g Polymerkomplex, bestehend aus Einheiten der Formel

$$NiSO_4\{(C_6H_5)_2P\text{-}(CH_2)_3SiO_{3/2}\cdot 20N[(CH_2)_3SiO_{3/2}]_3\cdot ZrO_2\}$$

erhalten.

| | |
|---|---|
| Kugelgröße ($d_{98\,\%}$) | 0,2 - 1,8 mm |
| Schüttdichte | 500 g/l |

| Elementaranalysen | % Ni | % P | % N | % Zr |
|---|---|---|---|---|
| Theorie | 0,9 | 0,48 | 4,3 | 1,4 |
| Gefunden | 0,8 | 0,50 | 4,0 | 1,3 |

Beispiel 14

Ausgehend von 3,0 g (10 mMol) $OsCl_3$, 217,3 g (500 mMol) $(C_6H_5)_2P\text{-}(CH_2)_3Si(OCH_3)_3$ sowie 251,9 g (500 mMol) $N[(CH_2)_3Si(OCH_3)_3]_3$ wurden unter Einsatz von 1-Hexanol anstelle von 2-Ethylhexanol sowie von Methanol anstelle von Ethanol und unter Praktizierung derselben Verfahrensweise wie in Beispiel 11, wobei allerdings die Vernetzerzugabe unterblieb, 288,0 g Polymerkomplex, bestehend aus Einheiten der Formel

$$OsCl_3\{(C_6H_5)_2P\text{-}(CH_2)_3SiO_{3/2}\cdot N[(CH_2)_3SiO_{3/2}]_3\}_{50}$$

erhalten.

| | |
|---|---|
| Kugelgröße ($d_{98\,\%}$) | 0,2 - 1,6 mm |
| Schüttdichte | 360 g/l |

| Elementaranalysen | % Os | % P | % N | % Si |
|---|---|---|---|---|
| Theorie | 0,65 | 5,3 | 2,4 | 19,3 |
| Gefunden | 0,6 | 5,1 | 2,2 | 18,6 |

Beispiel 15

Der Ansatz zur Herstellung des Polymerkomplexes

$$RhCl_3\{(C_6H_5)_2P\text{-}(CH_2))_3SiO_{3/2} \cdot N[(CH_2)_3SiO_{3/2}]_3\}_{20}$$

gemäß Beispiel 2 wurde wiederholt. Nach Abschluß der Rückflußphase und dem Erhalt des Xylol-feuchten geformten Rohproduktes wurde das 2-Phasensystem wie in Beispiel 2 in einen 3 l-Druckbehälter überführt. Auf den Druckbehälter wurden zunächst 50 bar CO und dann 50 bar $H_2$ aufgedrückt. Dann wurde die Mischung unter Rühren auf 140° C aufgeheizt und 30 Stunden bei dieser Temperatur gehalten. Anschließend wurde abgekühlt, entspannt und wie in Beispiel 2 aufgearbeitet. Nach dem Trocknen wurde das Produkt noch mit 3 l NaOH-Lösung (pH 12) und mit 2 1 Wasser gewaschen und erneut 12 Stunden bei 120° C getrocknet. Erhalten wurden 58,0 g eines geformten polymeren Rhodium-Komplexkatalysators, bestehend aus Polymereinheiten der Formel

$$RhH(CO)\{(C_6H_5)_2P\text{-}(CH_2)_3SiO_{3/2} \cdot N[(CH_2)_3SiO_{3/2}]_3\}_{20}.$$

| | |
|---|---|
| Kugelgröße ($d_{96\ \%}$) | 0,3 - 1,8 mm |
| Spezifisches Porenvolumen | 1,8 ml/g |
| Schüttdichte | 320 g/l |

| Elementaranalysen | % Rh | % Cl | % P | % N |
|---|---|---|---|---|
| Theorie | 0,88 | 0 | 5,3 | 2,4 |
| Gefunden | 0,8 | 0,15 | 5,2 | 2,3 |

| | |
|---|---|
| IR-Spektrum | Co ca. 1960 $cm^{-1}$ |
| | H ca. 2050 $cm^{-1}$ |

Beispiel 16

50 ml des in Beispiel 1 hergestellten Rh-haltigen Polymerkomplexes mit einer Korngröße von 0,3 bis 1,2 mm wurden in einen Rohrreaktor mit einem Innendurchmesser von 16 mm gefüllt. Der Rohrreaktor wurde in eine kontinuierliche Hydroformylierungsapparatur eingebaut. Nach der Inbetriebnahme der Anlage und nachdem nach 48 Stunden Betrieb konstante Bedingungen eingestellt waren, wurde die Hydroformylierung von Hexen-1 unter folgenden Bedingungen durchgeführt:

| | |
|---|---|
| Gesamtdruck | 120 bar |
| $H_2$/CO-Verhältnis | 1 : 1 |
| Temperatur im Reaktor | 100° C |
| Volumenstrom Hexen-1 | 50 ml/h |
| Gasstrom $H_2$/CO | 100 Nl/h |

Eine gaschromatische Analyse (GC-Analyse) des ausgetragenen und entspannten Produktes ergab eine Zusammensetzung von 98,6 % Gesamtaldehydgehalt (Rest: Olefinisomere, Octan) bei einem n : i-Produktverhältnis von 2. Der Rh-Gehalt des Produktes betrug weniger als 0,05 ppm. Nach 200, 400 und 600 Stunden Betriebsdauer wurden erneut GC-Analysen des Produktes durchgeführt. Dabei ergab sich eine angenähert gleiche Zusammensetzung, der Gehalt an Rhodium betrug ca. 30 ppb.

Beispiel 17

5,0 g des in Beispiel 4 hergestellten Pd-haltigen Polymerkomplexes mit einer Korngröße von 0,2 - 0,4 mm wurden

zusammen mit 234 g Vinylcyclohexen in einem 1 l-Autoklaven vereinigt. Auf den Autoklaven wurde ein konstanter Druck von 5 bar $H_2$ aufgegeben, wobei der verbrauchte Wasserstoff ständig aus einem Reservoir ergänzt wurde. Anschließend wurde unter Rühren (1000 U/Min.) auf 60° C aufgeheizt und solange weiter gerührt (ca. 3 Stunden), bis die theoretische Menge Wasserstoff, die für die Hydrierung einer Doppelbindung erforderlich ist, verbraucht war. Dann wurde abgekühlt und eine GC-Untersuchung der Produktmischung durchgeführt. Dieser Untersuchung zufolge waren ca. 88 % der eingesetzten Eduktmenge zu Ethylcyclohexen hydriert worden.

Beispiel 18

5,0 g des in Beispiel 7 hergestellten Ir-haltigen Polymerkomplexes mit einer Korngröße von 50 µm bis 0,1 mm wurden zusammen mit 166,2 g Tetrahydrobenzaldehyd in einem 1 l-Autoklaven vereinigt. Der Autoklav wurde mit 10 bar Wasserstoff beaufschlagt, wobei der verbrauchte Wasserstoff ständig aus einem Reservoir ergänzt wurde. Unter Rühren (1000 U/Min.) wurde auf 70° C aufgeheizt und solange weiter gerührt (ca. 4 Stunden), bis die theoretische Menge Wasserstoff, die für die Hydrierung einer Doppelbindung erforderlich ist, verbraucht war. Eine GC-Analyse des erhaltenen Produktes zeigte, daß 90 % des eingesetzten Eduktes zu Tetrahydrobenzylalkohol umgesetzt worden waren.

Beispiel 19

5 g des in Beispiel 10 hergestellten Pt-haltigen Polymerkomplexes mit einer Korngröße von 0,2 - 0,6 mm wurden zusammen mit 221,5 g Octen-1 und 267,3 g $HSiCl_3$ in einem 1 l-Glasautoklaven vereinigt. Unter Rühren (1000 U/Min.) wurde die Reaktionsmischung auf 100° C aufgeheizt und 20 Stunden bei dieser Temperatur gehalten. Eine GC-Analyse des erhaltenen Produkts zeigte, daß 95 % des eingesetzten Octen-1 zu Octyltrichlorsilan umgesetzt worden waren.

**Patentansprüche**

**1.** Kugelförmige polymere Komplexverbindungen des Eisens, Kobalts, Nickels, Rutheniums, Rhodiums, Palladiums, Osmiums, Iridiums und/oder Platins,
**dadurch gekennzeichnet,**
daß der Ligand aus einem geformten Organosiloxan-Copolykondensat besteht, das aus Einheiten der Formel

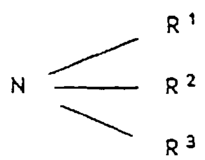

(I)

und aus Einheiten der Formel

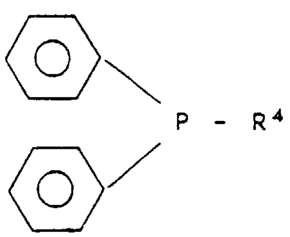

(II)

in einem Verhältnis von 10:90 bis 95:5 Mol % besteht, wobei das jeweilige Zentralatom über die bindungsstarken Phosphoratome der Phosphin-Einheiten (II) oder zusätzlich auch über die bindungsschwächeren Stickstoffatome der Amineinheiten (I) koordinativ gebunden ist, $R^2$ bis $R^4$ gleich oder verschieden sind und eine Gruppe der allgemeinen Formel

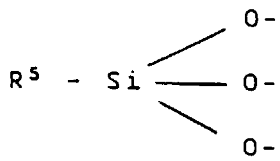 (III)

bedeuten, wobei $R^5$ direkt an das Phosphoratom bzw. an das Stickstoffatom gebunden ist und eine lineare oder verzweigte Alkylengruppe mit 1 bis 10 C-Atomen, eine Cyloalkylengruppe mit 5 bis 8 C-Atomen oder eine Einheit der allgemeinen Formel

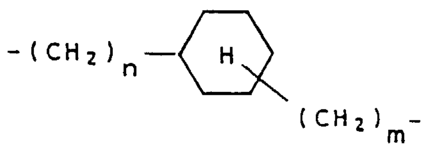 oder 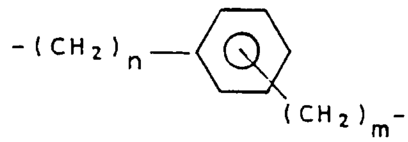

darstellt, in der n und m eine Zahl von 0 bis 6 ist, wobei n die Zahl N-ständiger bzw. P-ständiger und m die Zahl Si-ständiger Methylengruppen angibt, $R^1$ ebenfalls eine Gruppe der Formel (III) darstellt oder für H, $CH_3$, $C_2H_5$, $C_3H_7$ steht, wobei die freien Valenzen der an das Si-Atom gebundenen Sauerstoffatome wie bei Kieselsäuregerüsten durch Siliciumatome weiterer Gruppen der Formel (III) und/oder über die Metallatome in einem oder mehreren vernetzenden Brückengliedern

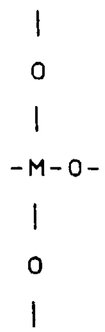 oder 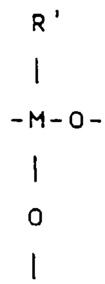 oder 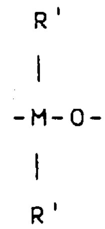

(IV)

mit M = Si, Ti, Zr bzw.

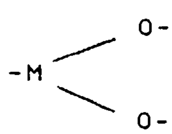 oder 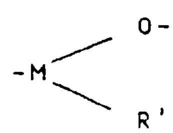

mit M = Al abgesättigt sind, und R' eine lineare oder verzweigte Alkylgruppe mit 1 bis 5 C-Atomen oder eine Phenylgruppe ist und das Verhältnis der Siliciumatome aus den Gruppen der allgemeinen Formel (III) zu den Metallatomen in den vernetzenden Brückengliedern (IV) 1 : 0 bis 1 : 20 und das molare Verhältnis an Phosphin-Einheiten (II) zu komplexierten Metalleinheiten 1 : 1 bis 100 : 1, beträgt und die polymeren Komplexkatalysatoren makroskopisch als kugelförmige Teilchen mit einem Durchmesser von 0,05 bis 2,0 mm, einer spezifischen Oberfläche von > 0 bis 1000 $m^2/g$, vorzugsweise > 0 bis 700 $m^2/g$, einem spezifischen Porenvolumen von 0.01 bis 6,5 ml/g, sowie einer Schüttdichte von 100 bis 700 g/l, vorliegen, wobei das komplexgebundene Metall homogen dispers, d.h. gleichmäßig über das geformte Partikel verteilt ist.

**2.** Geformte polymere Komplexverbindungen nach Anspruch 1,
**dadurch gekennzeichnet,**

daß $R^1$ bis $R^4$ eine Gruppe der allgemeinen Formel (III) sind und gleich oder verschieden sind.

3. Geformte polymere Komplexverbindungen nach den Ansprüchen 1 bis 2,
**dadurch gekennzeichnet,**
daß sie als statistische Copolykondensate, Block-Copolykondensate oder auch als gemischte Copolykondensate vorliegen.

4. Geformte polymere Komplexverbindungen nach den Ansprüchen 1 bis 3,
**dadurch gekennzeichnet,**
daß $R^1$ bis $R^4$ für eine Gruppe der allgemeinen Formel

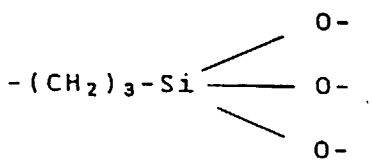

(V)

stehen.

5. Geformte polymere Komplexverbindungen nach den Ansprüchen 1 bis 4,
**dadurch gekennzeichnet,**
daß an die Polymereinheiten nach Formel (II) und Formel (I) eine oder mehrere Metalleinheiten (VI) von

$FeX_3$, $FeX_2$
$CoX_3$, $CoX_2$,
$NiX_2$,
$RuX_3$, $RuX_2$,
$RhX_3$, $RhX_2$, RhX, Rh(dien)X, RhXCO
$PdX_4$, $PdX_2$, $Pd^O$
$OsX_3$
$IrX_3$, IrX, Ir(dien)X, IrX(CO)
$PtX_4$, $PtX_2$, Pt°

gebunden sind,
wobei X für Cl, Br, J, 0,5 $SO_4$, H, Acetylacetonat, Acetat, $NO_3$, CN und dien für Cyclooctadien, Norbornadien steht.

6. Geformte polymere Komplexverbindungen nach den Ansprüchen 1 bis 5,
**dadurch gekennzeichnet,**
daß die Metalleinheiten nach Formel (VI) jeweils über mindestens eine Phosphineinheit nach Formel (II) an die Matrix gebunden sind.

7. Geformte polymere Komplexverbindungen nach den Ansprüchen 1 bis 6,
**dadurch gekennzeichnet,**
daß die Metalleinheiten nach Formel (VI) jeweils nur über Phosphineinheiten nach Formel (II) an die Polymermatrix gebunden sind.

8. Geformte polymere Komplexverbindungen nach den Ansprüchen 1 bis 7,
**dadurch gekennzeichnet,**
daß der Metallgehalt im Polymersystem mindestens 0,01 Gew.% und höchstens 20 Gew.%, vorzugsweise 0,1 Gew.% bis 10 Gew.%, beträgt.

9. Geformte polymere Komplexverbindungen nach den Ansprüchen 1 bis 8,
**dadurch gekennzeichnet,**
daß neben den die Metallzentren nach Formel (VI) komplexierenden Liganden nach Formeln (II) und (I) noch weitere überschüssige, nicht komplexierende Liganden nach Formeln (I) und/oder (II) im Polymersystem vorhanden sind.

**10.** Geformte polymere Komplexverbindungen nach den Ansprüchen 1 bis 9,
**dadurch gekennzeichnet,**
daß im Polymersystem nicht mehr Liganden nach Formel (II) vorliegen, als zum Aufbau des jeweiligen Metallkomplexes maximal benötigt werden, so daß das stöchiometrische Verhältnis zwischen den Liganden nach Formel (II) und dem Metall mindestens 1 : 1, aber, in Abhängigkeit vom jeweiligen Metall für Fe, Co, Rh, Pd, Pt, Ni höchstens 4 : 1 und für Ru, Os, Ir maximal 3 : 1 beträgt und daneben noch weitere Liganden nach Formel (I) im Polymersystem vorliegen.

**11.** Verfahren zur Herstellung der geformten polymeren Metallkomplexe nach den Ansprüchen 1 bis 10,
**dadurch gekennzeichnet,**
daß man eine oder mehrere wasserhaltige oder wasserfreie Metallverbindungen (VII) von

$FeX_3$, $FeX_2$
$CoX_3$, $CoX_2$
$NiX_2$
$RuX_3$, $RuX_3(CH_3CN)_3$, $RuX_3(C_6H_5CN)_3$
$M_3RhX_6$, $RhX_3$, $RhX_3(CH_3CN)_3$,
$RhX_3(C_6H_5CN)_3$, $RhX_2$,
$[RhX(dien)]_2$
$M_2PdX_6$, $M_2PdX_4$, $PdX_2$
$OsX_3$, $OsX_3(CH_3CN)_3$, $OsX_3(C_6H_5CN)_3$
$M_3IrX_6$, $IrX_3$, $IrX_3(CH_3CN)_3$,
$IrX_3(C_6H_5CN)_3$, $[IrX(dien)]_2$
$M_2PtX_6$, $M_2PtX_4$, $PtX_2$,

wobei

X = Cl, Br, J, Acetylacetonat, Acetat, $\frac{1}{2}$ $SO_4$, $NO_3$, CN
dien = Cyclooctadien, Norbornadien und
M = H, Na, K, $NH_4$,

in einem Lösungsmittel oder Lösungsmittelgemisch über einen Zeitraum von 1 Min. bis zu 48 Stunden mit einem Phosphin der allgemeinen Formel

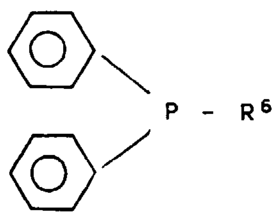

(VIII)

wobei $R^6$ eine Gruppe der allgemeinen Formel

$$R^5 - Si(OR^7)_3 \qquad (IX)$$

bedeuten, $R^5$ dieselbe Bedeutung wie in Formel (III) von Anspruch 1 hat, $R^7$ eine lineare oder verzweigte Alkylgruppe mit 1 bis 5 C-Atomen bedeutet und das Verhältnis zwischen der Molzahl an Phosphin nach Formel (VIII) und der Molzahl der insgesamt komplex gebundenen Metallatome in den Metallverbindungen nach Formel (VII) 1 : 1 bis 100 : 1 beträgt, zum Metallkomplex umsetzt, der erhaltenen Lösung anschließend ein Aminosilan der allgemeinen Formel

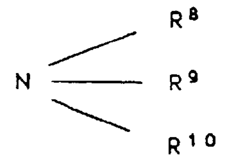 (X)

wobei $R^8$ für H, $CH_3$, $C_2H_5$, $C_3H_7$ oder eine Gruppe der allgemeinen Formel (IX) und $R^9$ sowie $R^{10}$ ebenfalls für eine Gruppe der Formel (IX) stehen, in der $R^5$ und $R^7$ denselben Bedeutungsumfang wie in Formel (IX) haben und gegebenenfalls eine oder mehrere Verbindungen der allgemeinen Formel

$$M(OR)_{2-4}\,R'_{0-2} \text{ bzw. } M(OR)_{2-3}\,R'_{0-1} \qquad \text{(XI)}$$

wobei M ein Si-, Ti-, Zr- oder Al-Atom, R' eine lineare oder verzweigte Alkylgruppe mit 1 bis 5 C-Atomen oder eine Phenylgruppe ist, R eine lineare oder verzweigte Alkylgruppe mit 1 bis 5 C-Atomen bedeutet und das Verhältnis der Siliciumatome aus den Gruppen der allgemeinen Formel (IX) zu den Metallatomen in den Vernetzern (XI) 1 : 0 bis 1 : 20 beträgt,
zusetzt, der erhaltenen Lösung unter Rühren eine zumindest für die vollständige Hydrolyse und Kondensation ausreichende Menge Wasser zufügt, das Reaktionsgemisch über einen Zeitraum bis zu 6 Stunden hydrolysiert, dann unter Weiterrühren unter der Maßgabe gelieren läßt, daß man bei Gelierungsbeginn oder bis zu einer Stunde danach mit 10 bis 2000, Gew.% bezogen auf die Gesamtmenge von Phosphin (VIII), Aminoorganosilan (X) und gegebenenfalls Vernetzer (XI), eines weitgehend wasserunlöslichen, aber die (an)gelierte Reaktionsmischung lösenden Lösungsmittels versetzt und homogenisiert, dem viskosen Homogenisat 10 bis 2000 Gew.%, bezogen auf die Gesamtmenge von Phosphin (VIII), Aminoorganosilan (X) und gegebenenfalls Vernetzer (XI), Wasser zugibt, die den polymeren Metallkomplex in gelierender Form enthaltende organische Phase in dem flüssigen Zweiphasensystem dispergiert und den sich in Form von Kugeln bildenden Feststoff von der flüssigen Phase abtrennt, dann gegebenenfalls mit einem niedrigsiedenden Lösungsmittel extrahiert, bei Raumtemperatur bis 250° C, gegebenenfalls unter Schutzgas oder im Vakuum trocknet und 1 bis 100 Stunden bei Temperaturen von 150° C bis 300° C tempert und/oder klassifiziert.

**12.** Verfahren nach Anspruch 11,
**dadurch gekennzeichnet,**
daß bei der Hydrolyse als Lösungsmittel Methanol, Ethanol, n- und i-Propanol, n- und i-Butanol oder n-Pentanol, allein oder in Mischung, verwendet wird.

**13.** Verfahren nach Anspruch 11 oder 12,
**dadurch gekennzeichnet,**
daß man die Hydrolyse mit Wasserüberschuß durchführt.

**14.** Verfahren nach den Ansprüchen 11 bis 13,
**dadurch gekennzeichnet,**
daß der (an)gelierten Reaktionsmischung ein linearer oder verzweigter Alkohol mit 4 bis 12 C-Atomen, Toluol, Ethylbenzol oder o-, m-, p-Xylol, oder Gemische davon zugesetzt werden.

**15.** Verfahren nach den Ansprüchen 11 bis 14,
**dadurch gekennzeichnet,**
daß die Gelierung und Formung bei Normaldruck oder einem Überdruck, welcher der Summe der Partialdrucke der Komponenten der Reaktionsmischung bei der jeweils angewandten Temperatur entspricht, durchgeführt wird.

**16.** Verfahren nach den Ansprüchen 11 bis 15,
**dadurch gekennzeichnet,**
daß man gemäß Anspruch 11 einen monomeren Metallkomplex in Lösung bildet, dieser gegebenenfalls einen Teil oder die vollständige Menge einer oder mehrerer der Verbindungen der allgemeinen Formel (XI) zusetzt, die Mischung in Gegenwart einer nicht zur vollständigen Hydrolyse ausreichenden Menge Wasser, über einen Zeitraum von 5 Min. bis zu 48 Stunden bei Raumtemperatur bis 200° C vorkondensiert, anschließend ein Aminosilan der Formel (X), gegebenenfalls die restliche Menge einer oder mehrerer der Verbindungen nach Formel (XI), gegebenenfalls weiteres Lösungsmittel und in jedem Fall weiteres Wasser zusetzt, erneut über einen Zeitraum bis zu 4 Stunden, hydrolysiert und anschließend weiter wie nach Anspruch 11 verfährt.

**17.** Verfahren nach Anspruch 16,
**dadurch gekennzeichnet,**
daß die Vorkondensation in Gegenwart eines sauren, basischen oder Metall-haltigen Kondensationskatalysators durchgeführt wird.

**18.** Verfahren gemäß Anspruch 16,
**dadurch gekennzeichnet,**
daß die Vorkondensation nur mit dem durch eine kristallwasserhaltige Metallkomponente eingebrachten Wasser durchgeführt wird.

**19.** Verfahren gemäß Anspruch 16 oder 17,
**dadurch gekennzeichnet,**
daß die zur Vorkondensation verwendete, über die gegebenenfalls vorhandene Kristallwassermenge hinausgehende Menge Wasser gleich zu Beginn der Umsetzung der Metallkomponente (VII) mit dem Phosphin (VIII) zugesetzt wird.

**20.** Verfahren zur Herstellung der geformten polymeren Metallkomplexe nach den Ansprüchen 1 bis 10,
**dadurch gekennzeichnet,**
daß man gemäß Anspruch 16 ein Vorkondensat aus dem monomeren Metallkomplex und Verbindungen gemäß Formel (XI) bildet, wobei das Verhältnis zwischen der Molzahl an Phosphineinheiten (VIII) und der Molzahl der insgesamt komplex gebundenen Metallatome von 1 : 1 bis x : 1, reicht und x die jeweils metallspezifische maximale Koordinationszahl im jeweiligen Metallkomplex darstellt, anschließend die über die maximale Koordinationszahl des Metalls hinausgehende Menge an Phosphin (VIII), gegebenenfalls die restliche oder vollständige Menge einer oder mehrerer Verbindungen (XI) sowie ein Aminosilan (X), gegebenenfalls weiteres Lösungsmittel und in jedem Fall weiteres Wasser zugibt, erneut über einen Zeitraum bis zu 4 Stunden, hydrolysiert und anschließend weiter wie nach Anspruch 11 verfährt.

**21.** Verfahren zur Herstellung der geformten polymeren Metallkomplexe nach den Ansprüchen 1 bis 10,
**dadurch gekennzeichnet,**
daß man den aus der Umsetzung der Metallverbindung (VII) mit der Phosphinkomponente (VIII) gemäß Ansprüchen 11 bzw. 20 erhaltenen monomeren Metallkomplex zusammen mit gegebenenfalls vorhandenem überschüssigem Phosphin (VIII) bei oder nach seiner Herstellung vorkondensiert und ein Aminosilan der Formel (X) sowie gegebenenfalls eine oder mehrere Verbindungen der Formel (XI) jeweils unabhängig voneinander in Gegenwart einer nicht zur vollständigen Hydrolyse ausreichenden Menge Wasser, über einen Zeitraum von 5 Min. bis zu 48 Stunden bei Raumtemperatur bis 200° C vorkondensiert, anschließend die einzeln vorkondensierten Komponenten vereinigt und dann nach Zusatz von so viel Wasser, daß mindestens die für eine vollständige Hydrolyse stöchiometrisch erforderliche Menge Wasser anwesend ist, und gegebenenfalls weiterem Lösungsmittel die vollständige Hydrolyse und Polykondensation sowie weitere Aufarbeitung gemäß Anspruch 11 durchführt.

**22.** Verfahren zur Herstellung der geformten polymeren Metallkomplexe nach den Ansprüchen 1 bis 10,
**dadurch gekennzeichnet,**
daß man eine wasserfreie Metallverbindung (VII) mit einer Phosphinkomponente (VIII) gemäß Ansprüchen 11 bzw. 20 umsetzt aber nicht vorkondensiert und, jeweils davon unabhänging, ein Aminosilan (X) sowie gegebenenfalls eine oder mehrere Verbindungen (XI), ohne oder unter Verwendung eines Lösungsmittels, in Gegenwart einer nicht zur vollständigen Hydrolyse ausreichenden Menge Wasser. über einen Zeitraum von 5 Min. bis zu 48 Stunden bei Raumtemperatur bis 200° C vorkondensiert, das nicht vorkondensierte metallhaltige Umsetzungsprodukt mit den beiden Vorkondensaten vereinigt und dann nach Zusatz weiteren Wassers und gegebenenfalls weiteren Lösungsmittels, so daß mindestens die für eine vollständige Hydrolyse und Polykondensation stöchiometrisch erforderliche Menge Wasser anwesend ist, die vollständige Hydrolyse und Polykondensation sowie weitere Aufarbeitung gemäß Anspruch 11 durchführt.

**23.** Verfahren zur Herstellung der geformten polymeren Metallkomplexe nach den Ansprüchen 1 bis 10,
**dadurch gekennzeichnet.**
daß man eine wasserhaltige oder wasserfreie Metallverbindung (VII) in vorzugsweise polarem Lösungsmittel mit einem Phosphin (VIII) in Gegenwart eines Aminosilans (X) sowie gegebenenfalls einer oder mehrerer Verbindungen (XI) über einen Zeitraum von 1 Min. bis zu 48 Stunden gemäß Ansprüchen 11 bzw. 20 umsetzt, der Lösung unter Rühren eine zumindest für die vollständige Hydrolyse und Kondensation ausreichende Menge Wasser zusetzt und dann weiter wie nach Anspruch 11 verfährt.

**24.** Verfahren nach Anspruch 23,
**dadurch gekennzeichnet,**
daß während oder nach der Umsetzung der Komponenten wie nach Anspruch 16 vorkondensiert wird.

**25.** Verfahren zur Herstellung der geformten polymeren Metallkomplexe, bei denen in Formel (VI) X = H ist oder das Metall komplexgebunden in nullwertiger Form vorliegt, nach den Ansprüchen 1 bis 10,
**dadurch gekennzeichnet**
daß der gemäß Ansprüchen 11 bis 24 primär hergestellte monomere Metallkomplex vor oder nach einer gegebenenfalls durchgeführten Vorkondensation einer Behandlung mit einem Reduktionsmittel, gegebenenfalls bei erhöhter Temperatur, über einen Zeitraum von 1 Min. bis zu 48 Stunden unterzogen wird und anschließend wie nach Anspruch 11 die weitere Hydrolyse, Polykondensation und Aufarbeitung durchgeführt wird.

**26.** Verfahren zur Herstellung der geformten polymeren Metallkomplexe, bei denen in Formel (VI) X = H ist oder das Metall komplexgebunden in nullwertiger Form vorliegt, nach den Ansprüchen 1 bis 10,
**dadurch gekennzeichnet,**
daß der gemäß Ansprüchen 11 bis 24 primär hergestellte monomere Metallkomplex zunächst hydrolysiert und unter Formung polykondensiert wird und vor oder nach mindestens einer der gemäß Anspruch 11 vorgesehenen Aufarbeitungsstufen, suspendiert in Wasser oder einem Lösungsmittel. einer Reduktionsbehandlung nach Anspruch 25, gegebenenfalls unter Überdruck, unterworfen wird.

**27.** Verfahren zur Nachbehandlung der nach einem oder mehreren der Ansprüche 11 bis 26 erhaltenen aber nicht getrockneten geformten polymeren Metallkomplexe,
**dadurch gekennzeichnet,**
daB der noch Lösungsmittel- und wasserfeuchte Komplex in Anwesenheit von Wasser sowie gegebenenfalls einem wassermischbaren Lösungsmittel oder der im Herstellungsgang zuletzt vorliegenden Flüssigkeit als solche oder in Dampfform einer Temperaturbehandlung während 1 Stunde bis zu einer Woche bei 50 bis 300° C, vorzugsweise 100 bis 200°C, gegebenenfalls unter Überdruck, gegebenenfalls unter einer gleichzeitigen Reduktionsbehandlung gemäß Anspruch 26, bevorzugt in Wasserstoffatmosphäre und/oder mit Natriumborhydrid, unterworfen wird.

**28.** Verfahren gemäß Anspruch 27,
**dadurch gekennzeichnet,**
daß die Nachbehandlung in Gegenwart eines sauren, basischen oder Metall-haltigen Hydrolyse- bzw. Kondensationskatalysators durchgeführt wird.

**29.** Verwendung der geformten polymeren Metallkomplexe nach den Ansprüchen 1 bis 10 als Katalysatoren zur Durchführung von Hydroformylierungs-, Hydrierungs-, Oligomerisierungs-, Carbonylierungs-, Hydrosilylierungs-, Carboximethylierungs-, Isomerisierungsreaktionen sowie für Reaktionen von CO oder $CO_2$ mit $H_2$.

**30.** Verwendung nach Anspruch 29,
**dadurch gekennzeichnet,**
daß die geformten polymeren Metallkomplexe in Suspension oder im Festbett oder im Fließbett für Reaktionen in flüssiger oder gasförmiger Phase eingesetzt werden.

## Claims

**1.** Spherical, polymeric, complex compounds of iron, cobalt, nickel, ruthenium, rhodium, palladium, osmium, iridium and/or platinum, characterised in that the ligand consists of a shaped organosiloxane copolycondensate consisting of units of the formula

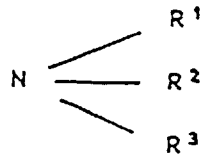

(I)

and of units of the formula

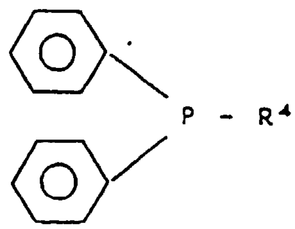

(II)

in a ratio of 10:90 to 95:5 mol.%, wherein the particular central atom forms a coordinate bond to the strong bonding phosphorus atoms in the phosphine units (II) or in addition also to the weaker bonding nitrogen atoms in the amine units (I), $R^2$ to $R^4$ are identical or different and represent a group of the general formula,

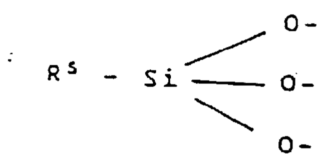

(III)

wherein $R^5$ is directly bonded to the phosphorus atom or nitrogen atom and represents a linear or branched alkylene group with 1 to 10 carbon atoms, a cycloalkylene group with 5 to 8 carbon atoms or a unit of the general formula,

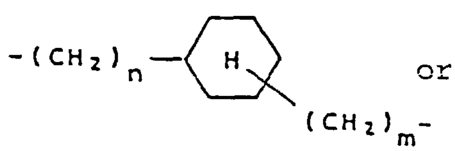

or

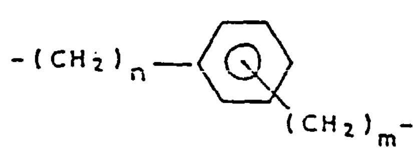

in which n and m represent a number from 0 to 6, wherein n gives the number of methylene groups adjacent to nitrogen or phosphorus and m gives the number of methylene groups adjacent to silicon, $R^1$ also represents a group of the formula (III) or H, $CH_3$, $C_2H_5$, $C_3H_7$, wherein the free valences on the oxygen atoms bonded to the silicon atom are saturated by silicon atoms from further groups of the formula (III), as in silicic acid structures, and/or via the metal atoms in one or more cross-linking bridge members

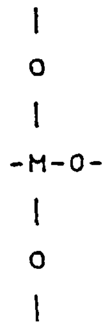 or 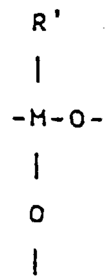 or 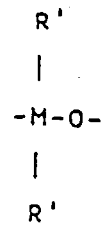

(IV)

where M = Si, Ti, Zr
or

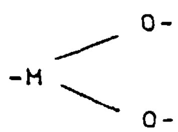 or 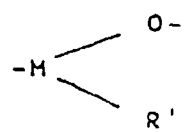

where M = Al, and $R^1$ is a linear or branched alkyl group with 1 to 5 carbon atoms or a phenyl group and the ratio of silicon atoms from the groups of the general formula (III) to metal atoms in the cross-linking bridging members (IV) is 1 : 0 to 1 : 20 and the molar ratio of phosphine units (II) to complexed metal units is 1 : 1 to 100 : 1, and the polymeric complex catalysts are present macroscopically as spherical particles with a diameter of 0.05 to 2.0 mm, and have a specific surface area of >0 to 1000 $m^2/g$, preferably >0 to 700 $m^2/g$, a specific pore volume of 0.01 to 6.5 ml/g, and a bulk density of 100 to 700 g/l, wherein the complex bonded metal is dispersed homogeneously, i. e. uniformly, over the shaped particle.

**2.** Shaped, polymeric, complex compounds according to Claim 1, characterised in that $R^1$ to $R^4$ represent a group of the general formula (III) and are identical or different.

**3.** Shaped, polymeric, complex compounds according to Claims 1 to 2, characterised in that they are present as statistical copolycondensates, block copolycondensates or as mixed copolycondensates.

**4.** Shaped, polymeric, complex compounds according to Claims 1 to 3, characterised in that $R^1$ to $R^4$ represent a group of the general formula.

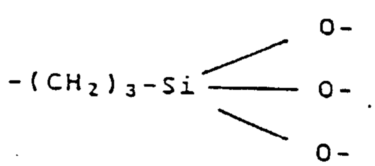

(V)

**5.** Shaped, polymeric, complex compounds according to Claims 1 to 4, characterised in that one or more metal units (VI) from

$FeX_3$, $FeX_2$
$CoX_3$, $CoX_2$
$NiX_2$
$RuX_3$, $RuX_2$

$RhX_3$, $RhX_2$, RhX, Rh(dien)X, RhXCO
$PdX_4$, $PdX_2$, $Pd^0$
$OsX_3$
$IrX_3$, IrX, Ir(dien)X, IrX(CO)
$PtX_4$, $PtX_2$, $Pt^0$

are bonded to the polymeric units according to formula (II) and formula (I), wherein X represents Cl, Br, I, 0.5 $SO_4$, H, acetylacetonate, acetate, $NO_3$, CN and dien represents cyclooctadiene or norbornadiene.

**6.** Shaped, polymeric, complex compounds according to Claims 1 to 5, characterised in that the metal units according to formula (VI) are each bonded to the matrix via at least one phosphine unit according to formula (II).

**7.** Shaped, polymeric, complex compounds according to Claims 1 to 6, characterised in that the metal units according to formula (VI) are each bonded to the polymer matrix only via phosphine units according to formula (II).

**8.** Shaped, polymeric, complex compounds according to Claims 1 to 7, characterised in that the metal content in the polymer system is at least 0.01 wt.% and at most 20 wt.%, preferably 0.1 wt.% to 10 wt.%.

**9.** Shaped, polymeric, complex compounds according to Claims 1 to 8, characterised in that, in addition to the ligands according to formulae (II) and (I) complexing the metal centres according to formula (VI), further excess, non-complexing ligands according to formulae (I) and/or (II) are present in the polymer system.

**10.** Shaped, polymeric, complex compounds according to Claims 1 to 9, characterised in that no more ligands according to formula (II) are present in the polymer system than are required as a maximum to build up the particular metal complex, so that the stoicheiometric ratio between the ligands according to formula (II) and the metal is at least 1:1 but, depending on the particular metal, is at most 4:1 for Fe, Co, Rh, Pd, Pt, Ni and at most 3:1 for Ru, Os, Ir and in addition still further ligands according to formula (I) are present in the polymer system.

**11.** Process for preparing shaped,- polymeric metal complexes according to Claims 1 to 10, characterised in that one or more hydrated or anhydrous metal compounds (VII) from

$FeX_3$, $FeX_2$
$CoX_3$, $CoX_2$
$NiX_2$
$RuX_3$, $RuX_3(CH_3CN)_3$, $RuX_3(C_6H_5CN)_3$
$M_3RhX_6$, $RhX_3$, $RhX_3(CH_3CN)_3$
$RhX_3(C_6H_5CN)_3$, $RhX_2$
$[RhX(dien)]_2$
$M_2PdX_6$, $M_2PdX_4$, $PdX_2$
$OsX_3$, $OsX_3(CH_3CN)_3$, $OsX_3$ $(C_6H_5CN)_3$
$M_3IrX_6$, $IrX_3$, $IrX_3(CH_3CN)_3$,
$IrX_3(C_6H_5CN)_3$, $[IrX(dien)]_2$
$M_2PtX_6$, $M_2PtX_4$, $PtX_2$

wherein

X = Cl, Br, I, acetylacetonate, acetate, 0.5 $SO_4$, $NO_3$, CN,
dien = cyclooctadiene, norbornadiene and
M = H, Na, K, $NH_4$,

in a solvent or solvent mixture is reacted over a period of 1 minute to 48 hours with a phosphine of the general formula

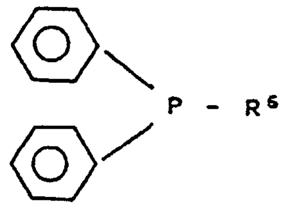

(VIII)

wherein $R^6$ represents a group of the general formula

$$R^5 - Si(OR^7)_3 \qquad (IX)$$

$R^5$ is defined in the same way as for formula (III) in Claim 1, $R^7$ represents a linear or branched alkyl group with 1 to 5 carbon atoms and the ratio between the number of moles of phosphine according to formula (VIII) and the number of moles of total metal atoms complex-bonded in the metal compounds according to formula (VII) is 1:1 to 100:1, to give a metal complex; an aminosilane of the general formula

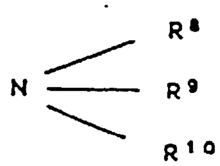

(X)

wherein $R^8$ represents H, $CH_3$, $c_2H_5$, $C_3H_7$ or a group of the general formula (IX) and $R^9$ and $R^{10}$ also represent a group of the formula (IX), in which $R^5$ and R7 are defined in the same way as in formula (IX) and optionally one or more compounds of the general formula

$$M(OR)_{2-4}\,R'_{0-2} \text{ or } M(OR)_{2-3}\,R'_{0-1} \qquad (XI)$$

wherein M is a Si, Ti, Zr or Al atom, R' is a linear or branched alkyl group with 1 to 5 carbon atoms or a phenyl group, R is a linear or branched alkyl group with 1 to 5 carbon atoms and the ratio of silicon atoms from groups of the general formula (IX) to metal atoms in the cross-linking agent (XI) is 1:0 to 1:20, at least enough water for complete hydrolysis and condensation is then added to the solution obtained, with stirring, the reaction mixture is hydrolysed for a period of up to 6 hours, then with further stirring can be gelled, with the proviso that at the start of the gelling procedure, or up to one hour after, that 10 to 2000 wt.%, with respect to the total amount of phosphine (VIII), aminoorganosilane (X) and optional cross-linking agent (XI), of a largely water-insoluble solvent, but one which can dissolve the gelled or gelling reaction mixture, is added and the mixture is homogenised, 10 to 2000 wt.%, with reference to the total amount of phosphine (VIII), aminoorganosilane (X) and optional cross-linking agent (XI), of water is added to the viscous homogenised material, the organic phase containing the polymeric metal complex in a gelling form is dispersed in the liquid two-phase system and the solid being produced in the form of spheres is separated from the liquid phase, then optionally extracted with a low boiling solvent, dried at room temperature to 250°C, optionally under a protective gas or under reduced pressure and maintained at a constant temperature of 150°C to 300°C for 1 to 100 hours and/or classified.

**12.** Process according to Claim 11, characterised in that methanol, ethanol, n- and i-propanol, n- and i-butanol or n-pentanol, alone or as a mixture are used as solvent during hydrolysis.

**13.** Process according to Claim 11 or 12, characterised in that the hydrolysis is performed with excess water.

**14.** Process according to Claims 11 to 13, characterised in that a linear or branched alcohol with 4 to 12 carbon atoms, toluene, ethylbenzene or o-, m-, p-xylene or mixtures thereof are added to the gelled or gelling reaction mixture.

**15.** Process according to Claims 11 to 14, characterised in that gelling and shaping are performed at atmospheric pressure or at a higher pressure which corresponds to the sum of the partial pressures of the components in the

reaction mixture at the particular temperature used.

**16.** Process according to Claims 11 to 15, characterised in that a monomeric metal complex is formed in solution in accordance with Claim 11, some or the whole amount of one or more compounds of the general formula (XI) are optionally added to this, the mixture is precondensed in the presence of an amount of water which is not sufficient for complete hydrolysis for a period of 5 minutes to 48 hours at room temperature to 200°C, then an aminosilane of the formula (X), optionally the remaining amount of one or more of the compounds according to formula (XI), optionally further solvent, and in any case further water, are added, hydrolysis is performed again for a period of up to 4 hours and then the same procedure is used as described in Claim 11.

**17.** Process according to Claim 16, characterised in that precondensation is performed in the presence of an acid, basic or metal-containing condensation catalyst.

**18.** Process according to Claim 16, characterised in that precondensation is performed only with the water introduced by a metal component which contains water of crystallisation.

**19.** Process according to Claim 16 or 17, characterised in that the amount of water over and above the optionally present amount of water of crystallisation is added right at the beginning of the reaction of metal component (VII) with phosphine (VIII).

**20.** Process for preparing shaped, polymeric, metal complexes according to Claims 1 to 10, characterised in that a precondensate is formed from the monomeric metal complex and compounds in accordance with formula (XI), in accordance with Claim 16, wherein the ratio between the number of moles of phosphine units (VIII) and the number of moles of total complex-bonded metal atoms extends from 1:1 to x:1 where x represents the particular metal-specific maximum coordination number in the particular metal complex, then the amount of phosphine (VIII) over and above the maximum coordination number of the metal, optionally the remaining or complete amount of one or more compounds (XI) and an aminosilane (X), optionally further solvent, and in any case further water, are added, hydrolysis is performed again for a period of up to 4 hours and then the same procedure is used as described in Claim 11.

**21.** Process for preparing shaped, polymeric, metal complexes according to Claims 1 to 10, characterised in that the monomeric metal complex obtained from reacting metal compound (VII) with the phosphine component (VIII) in accordance with Claims 11 or 20 is precondensed together with optionally present excess phosphine (VIII) during or after its preparation and an aminosilane of the formula (X) and optionally one or more compounds of the formula (XI), independently of each other, in the presence of an amount of water not sufficient for complete hydrolysis, is each precondensed for a period of 5 minutes to 48 hours at room temperature to 200°C, then the individual precondensed components are combined and then, after adding enough water so that at least the amount of water stoicheiometrically required for complete hydrolysis is present, and optionally further solvent, complete hydrolysis and polycondensation as well as further processing in accordance with Claim 11 are performed.

**22.** Process for preparing shaped, polymeric, metal complexes according to Claims 1 to 10, characterised in that an anhydrous metal compound (VII) is reacted but not precondensed with a phosphine component (VIII) in accordance with Claims 11 and 20 and, independently of that procedure, an aminosilane (X) and optionally one or more compounds (XI), with or without using a solvent, in the presence of an amount of water not sufficient for complete hydrolysis, each is precondensed for a period of 5 minutes to 48 hours at room temperature to 200°C, the non-precondensed metal-containing reaction product is combined with the two precondensates and, after adding further water and optionally further solvent so that at least the stoicheiometrically required amount of water for complete hydrolysis and polycondensation is present, complete hydrolysis and polycondensation as well as further processing in accordance with Claim 11 is performed.

**23.** Process for preparing shaped, polymeric, metal complexes according to Claims 1 to 10, characterised in that a hydrated or anhydrous metal compound (VII) in a preferably polar solvent is reacted with a phosphine (VIII) in the presence of an aminosilane (X) and optionally one or more compounds (XI) for a period of 1 minute to 48 hours in accordance with Claims 11 and 20, an amount of water at least sufficient for complete hydrolysis and condensation is added to the solution with stirring and the mixture is then processed further as described in Claim 11.

**24.** Process according to Claim 23, characterised in that precondensation according to Claim 16 is performed during or after reaction of the components.

**25.** Process for preparing shaped, polymeric, metal complexes, in which, in formula (VI), X = H or the metal is present complex-bonded in a zero-valent form, according to Claims 1 to 10, characterised in that the primary monomeric metal complex prepared in accordance with Claims 11 to 24 is subjected to treatment with a reducing agent, before or after an optionally performed precondensation process, optionally at elevated temperature, for a period of one minute to 48 hours and then further hydrolysis, polycondensation and processing are performed as described in Claim 11.

**26.** Process for preparing shaped, polymeric, metal complexes in which, in formula (VI), X = H or the metal is present complex-bonded in the zero-valent form, according to Claims 1 to 10, characterised in that the primary monomeric metal complex prepared according to Claims 11 to 24 is first hydrolysed and polycondensed with shaping and, before-or after at least one of the processing stages provided according to Claim 11, is suspended in water or a solvent and subjected to a reduction treatment in accordance with Claim 25, optionally at elevated pressure.

**27.** Process for after-treating the shaped but not dried, polymeric, metal complexes obtained by one or more of Claims 11 to 26 , characterised in that the complex which still contains solvent and moisture is subjected to a thermal treatment for from one hour up to one week at 50° to 300°C, preferably 100° to 200°C, optionally at elevated pressure, optionally with a simultaneous reduction treatment in accordance with Claim 26, preferably in a hydrogen atmosphere and/or with sodium borohydride, in the presence of water and optionally a water-miscible solvent or liquid present in the-previous preparation stage, as such or in vapour form.

**28.** Process according to Claim 27, characterised in that the after-treatment is performed in the presence of an acid, basic or metal-containing hydrolysis or condensation catalyst.

**29.** Use of the shaped, polymeric metal complexes according to Claims 1 to 10 as catalysts for performing hydroformylation, hydrogenation, oligomerisation, carbonylisation, hydrosilylisation, carboxymethylisation or isomerisation reactions and for reactions of CO or $CO_2$ with $H_2$.

**30.** Use according to Claim 29, characterised in that the shaped, polymeric metal complexes are used in suspension or in a fixed bed or in a fluidised bed for reactions in the liquid or gaseous phase.

**Revendications**

**1.** Composés complexes polymères sphériques du fer, du cobalt, du nickel, du ruthénium, du rhodium, du palladium, de l'osmium, de l'iridium et/ou du platine, caractérisés en ce que le ligand est constitué par un condensat de copolymère d'organosiloxane moulé, constitué par des unités de formule

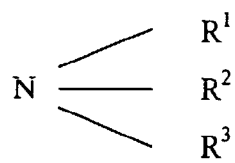

(I)

et par des unités de formule

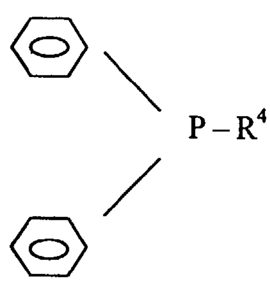

(II)

dans un rapport compris entre 10 :90 et 95 :5 mole-%, chaque atome central étant relié par coordination via les atomes de phosphore à fort pouvoir de liaison des unités phosphine (II) ou-en outre également via les atomes d'azote dont le pouvoir de liaison est moindre des unités amine (I),

$R^2$ à $R^4$ étant identiques ou différents et représentant un groupe de formule générale

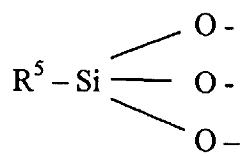

(III)

$R^5$ étant lié directement à l'atome de phosphore ou à l'atome d'azote et représentant un groupe alkylène linéaire ou ramifié comportant 1 à 10 atomes de carbone, un groupe cycloalkylène comportant 5 à 8 atomes de carbone ou une unité de formule générale

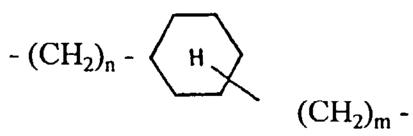

ou

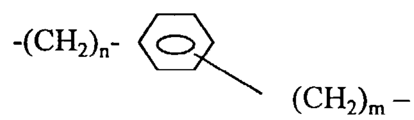

dans lesquelles n et m représentent un nombre de 0 à 6, n indiquant les groupes méthylène en position N ou en position P et m le nombre de groupes méthylène sur le Si, $R^1$ représentant également un groupe de formule (III) ou le reste H, $CH_3$, $C_2H_5$, $C_3H_7$, les valences libres des atomes d'oxygène liés à l'atome de Si étant saturées, comme dans les structures de silice, par des atomes de silicium d'autres groupes de formule (III) et/ou via les atomes métalliques dans un ou plusieurs éléments de pont de réticulation

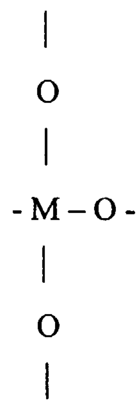

ou

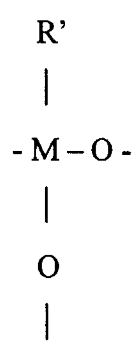

ou

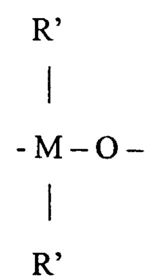

(IV)

avec M = Si, Ti, Zr, ou

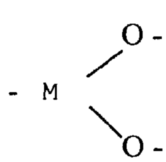

ou

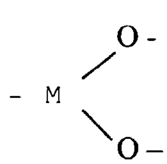

avec M = Al

et R' représentant un groupe alkyle linéaire ou ramifié comportant 1 à 5 atomes de carbone ou un groupe

phényle et le rapport entre les atomes de silicium des groupes de formule générale (III) et les atomes de métal dans les éléments de pont de réticulation (IV) étant compris entre 1 :0 et 1 :20 et le rapport molaire entre les unités phosphine (II) et les unités métalliques complexées étant compris entre 1 :1 et 100 :1 et les catalyseurs complexes polymères se trouvant à l'échelle macroscopique sous forme de particules sphériques présentant un diamètre de 0,05 à 2,0 mm, une surface spécifique de >0 à 1000 $m^2/g$, de préférence de >0 à 700 $m^2/g$, un volume spécifique des pores de 0,01 à 6,5 ml/g ainsi qu'une densité apparente de 100 à 700 g/l, le métal lié par complexation étant réparti par une dispersion homogène, c'est-à-dire régulièrement, entre les particules moulées.

**2.** Composés complexes polymères moulés selon la revendication 1, caractérisés en ce que $R^1$ à $R^4$ représentent un groupe de formule général (III) et sont identiques ou différents.

**3.** Composés complexes polymères moulés selon les revendications 1 à 2, caractérisés en ce qu'ils se présentent sous forme de condensats de copolymères statistiques, de condensats de copolymères en blocs ou également sous forme de condensats de copolymères mixtes.

**4.** Composés complexes polymères moulés selon les revendications 1 à 3, caractérisés en ce que $R^1$ à $R^4$ représentent un groupe de formule générale :

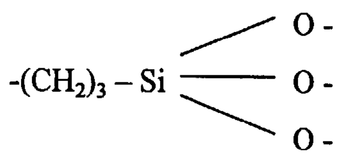

(V)

**5.** Composés complexes polymères moulés selon les revendications 1 à 4, caractérisés en ce qu'une ou plusieurs unités de métal (VI) de

$FeX_3$, $FeX_2$
$CoX_3$, $CoX_2$,
$NiX_2$,
$RuX_3$, $RuX_2$,
$RhX_3$, $RhX_2$, RhX, Rh(diène)X, RhXCO
$PdX_4$, $PdX_2$, $Pd^0$
$OsX_3$
$IrX_3$, IrX , Ir(diène)X, IrX(CO)
$PtX_4$, $PrX_2$, $Pt^0$

sont reliées aux unités de polymère selon la formule (II) et la formule (I), X représentant un reste Cl, Br, I, 0,5 $SO_4$, H, acétonate d'acétyle, acétate, $NO_3$, CN et diène représentant un reste cyclooctadiène, norbornadiène.

**6.** Composés complexes polymères moulés selon les revendications 1 à 5, caractérisés en ce que les unités de métal selon la formule (VI) sont chaque fois reliées à la matrice via au moins une unité phosphine selon la formule (II).

**7.** Composés complexes polymères moulés selon les revendications 1 à 6, caractérisés en ce que les unités de métal selon la formule (II) ne sont reliées à la matrice de polymère que via des unités phosphine selon la formule (VI).

**8.** Composés complexes polymères moulés selon les revendications 1 à 7, caractérisés en ce que la teneur en métal dans le système de polymères est comprise entre au moins 0,01 % en poids et au plus de 20 % en poids, de préférence entre 0,1 % en poids et 10 % en poids.

**9.** Composés complexes polymères moulés selon les revendications 1 à 8, caractérisés en ce que, en plus des ligands selon les formules (II) et (I) complexant les centres métalliques selon la formule (VI), on trouve encore d'autres ligands non complexants en excès selon les formules (I) et/ou (II) dans le système de polymères.

**10.** Composés complexes polymères moulés selon les revendications 1 à 9, caractérisés en ce qu'on ne trouve pas, dans le système de polymères, plus de ligands selon la formule (II) que le nombre maximal nécessaire pour la construction du complexe métallique, de telle manière que le rapport stoechiométrique entre les ligands de formule (II) et le métal est d'au moins 1 :1 et, en fonction du métal, d'au plus 4 :1 pour le Fe, Co, Rh, Pd, Pt, Ni et d'au plus 3 :1 pour le Ru, Os, Ir et qu'on trouve entre outre encore d'autres ligands selon la formule (I) dans le système de polymères.

**11.** Procédé pour la fabrication des complexes métalliques polymères moulés selon les revendications 1 à 10, caractérisé en ce qu'on fait réagir, pour obtenir un complexe métallique, un ou plusieurs composés métalliques (VII) contenant de l'eau ou exempts d'eau de

$FeX_3$, $FeX_2$
$CoX_3$, $CoX_2$,
$NiX_2$,
$RuX_3$, $RuX_3(CH_3CN)_3$, $RuX_3(C_6H_5CN)_3$
$M_3RhX_6$, $RhX_3$, $RhX_3(CH_3CN)_3$,
$RhX_3(C_6H_5CN)_3$, $RhX_2$,
$[RhX(\text{diène})]_2$
$M_2PdX_6$, $M_2PdX_4$, $PdX_2$
$OsX_3$, $OsX_3(CH_3CN)_3$, $OsX_3(C_6H_5CN)_3$
$M_3IrX_6$, $IrX_3$, $IrX_3(CH_3CN)_3$,
$IrX_3(C_6H_5CN)_3$, $[IrX(\text{diène})]_2$
$M_2PtX_6$, $M_2PtX_4$, $PtX_2$,

où

X = Cl, Br, I, acétonate d'acétyle, acétate, ½ $SO_4$, $NO_3$, CN
diène = cyclooctadiène, norbornadiène et
M = H, $N_a$, K, $NH_4$,

dans un solvant ou un mélange de solvants, pendant un laps de temps compris entre 1 minute et 48 heures, avec une phosphine de formule générale

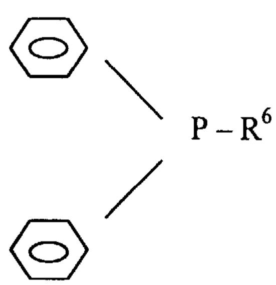 (VIII)

$R^6$ représentant un groupe de formule générale

$$R^5 - Si(OR^7)_3 \qquad \text{(IX)},$$

$R^5$ ayant la même signification que dans la formule (III) de la revendication 1, $R^7$ représentant un groupe alkyle linéaire ou ramifié comportant 1 à 5 atomes de carbone et le rapport entre le nombre molaire en phosphine selon la formule (VIII) et le nombre molaire de la totalité des atomes métalliques liés par complexation dans les composés métalliques selon la formule (VII) étant compris entre 1 :1 et 100 :1, en ce qu'on ajoute ensuite à la solution obtenue un aminosilane de formule générale

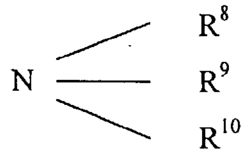

(X)

$R^8$ représentant H, $CH_3$, $C_2H_5$, $C_3H_7$ ou un groupe de formule générale (IX), et $R^9$ ainsi que $R^{10}$ représentant également un groupe de formule (IX), dans laquelle $R^5$ et $R^7$ ont la même signification que dans la formule (IX) et représentent, le cas échéant, un ou plusieurs composés de formule générale

$$M(OR)_{2\text{-}4}\,R'_{0\text{-}2} \text{ ou } M(OR)_{2\text{-}3}R'_{0\text{-}1} \qquad (XI)$$

M représentant un atome de Si, de Ti, de Zr ou d'Al, R' un groupe alkyle linéaire ou ramifié comportant 1 à 5 atomes de carbone ou un groupe phényle, R un groupe alkyle linéaire ou ramifié comportant 1 à 5 atomes de carbone et le rapport entre les atomes de silicium des groupes de formule générale (IX) et les atomes métalliques dans des éléments de réticulation (XI) étant compris entre 1 :0 et 1 :20, en ce qu'on ajoute à la solution obtenue, sous agitation, une quantité d'eau au moins suffisante pour l'hydrolyse et la condensation complètes, en ce qu'on hydrolyse le mélange réactionnel pendant un laps de temps allant jusqu'à 6 heures, en ce qu'on laisse gélifier en continuant l'agitation, sous réserve d'ajouter et d'homogénéiser, à partir du début de la gélification ou jusqu'à une heure après le début de celle-ci, 10 à 2000 % en poids, par rapport à la quantité totale de phosphine (VIII), d'aminoorganosilane (X) et le cas échéant, d'agent de réticulation (XI), d'un solvant dans une large mesure non soluble dans l'eau, mais solubilisant le mélange réactionnel gélifié ou en cours de gélification, en ce qu'on ajoute au produit d'homogénéisation visqueux 10 à 2000 % en poids par rapport à- la quantité totale de phosphine (VIII), d'aminoorganosilane (X) et le cas échéant, d'agent de réticulation (XI), d'eau qui disperse la phase organique contenant le complexe métallique polymère sous forme gélifiée dans le système liquide à deux phases et en ce qu'on sépare le solide qui se forme à l'état de billes de la phase liquide, en ce qu'on extrait, le cas échéant, avec un solvant à point d'ébullition bas, en ce qu'on sèche entre la température ambiante et 250 °C, le cas échéant sous un gaz protecteur ou sous vide, et en ce qu'on traite thermiquement et/ou classifie pendant 1 à 100 heures à des températures comprises entre 150 et 300 °C.

**12.** Procédé selon la revendication 11, caractérisé en ce qu'on utilise comme solvant au cours de l'hydrolyse du méthanol, de l'éthanol, du n- et du i-propanol, du n-et du i-butanol ou du n-pentanol, seuls ou en mélange.

**13.** Procédé selon la revendication 11 ou 12, caractérisé en ce qu'on réalise l'hydrolyse avec un excès d'eau.

**14.** Procédé selon les revendications 11 à 13, caractérisé en ce qu'on ajoute au mélange réactionnel gélifié ou en cours de gélification, un alcool linéaire ou ramifié comportant 4 à 12 atomes de carbone, du toluène, de l'éthylbenzène ou du o-, m-, p-xylène ou un mélange de ceux-ci.

**15.** Procédé selon les revendications 11 à 14, caractérisé en ce que la gélification et le moulage sont réalisés à la pression normale ou sous une surpression correspondant à la somme des pressions partielles des composants du mélange réactionnel à la température utilisée.

**16.** Procédé selon les revendications 11 à 15, caractérisé en ce qu'on forme, selon la revendication 11, un complexe métallique monomère en solution, en ce qu'on y ajoute, le cas échéant, une partie ou la quantité totale d'un ou de plusieurs des composés de formule générale (XI), en ce qu'on précondense le mélange en présence d'une quantité d'eau qui n'est pas suffisante pour une hydrolyse complète sur un laps de temps compris entre 5 min. et 48 heures entre la température ambiante et 200 °C, en ce qu'on ajoute ensuite un aminosilane de formule (X), le cas échéant la quantité résiduelle d'un ou de plusieurs composés selon la formule (XI), le cas échéant un autre solvant et, dans tous les cas, encore de l'eau, en ce qu'on hydrolyse à nouveau pendant un laps de temps allant jusqu'à 4 heures et en ce qu'on procède ensuite comme dans la revendication 11.

**17.** Procédé selon la revendication 16, caractérisé en ce qu'on réalise la précondensation en présence d'un catalyseur de condensation acide, basique ou contenant un métal.

**18.** Procédé selon la revendication 16, caractérisé en ce que la précondensation n'est réalisée qu'avec l'eau introduite par un composant métallique contenant de l'eau de cristallisation.

**19.** Procédé selon la revendication 16 ou 17, caractérisé en ce qu'on ajoute la quantité d'eau utilisée pour la précondensation, supérieure à la quantité d'eau de cristallisation, qui est le cas échéant présente, directement au début de la réaction des composants métalliques (VII) avec la phosphine (VIII).

**20.** Procédé pour la fabrication des complexes métalliques polymères moulés selon les revendications 1 à 10, caractérisé en ce qu'on forme, selon la revendication 16, un précondensat à partir du complexe métallique monomère et les composés selon la formule (XI), le rapport entre le nombre molaire d'unités phosphine (VIII) et le nombre molaire de la totalité des atomes métalliques liés par complexation étant compris entre 1 :1 et x :1, et x représentant chaque fois le nombre de coordination maximal spécifique à chaque métal dans le complexe métallique, en ce qu'on ajoute ensuite la quantité de phosphine (VIII) supérieure au nombre de coordination maximal du métal, le cas échéant la quantité résiduelle ou complète d'un ou de plusieurs composés (XI) ainsi qu'un aminosilane (X), le cas échéant un autre solvant et dans tous les cas de l'eau supplémentaire, en ce qu'on hydrolyse à nouveau pendant un laps de temps allant jusqu'à 4 heures et en ce qu'on traite ensuite comme dans la revendication 11.

**21.** Procédé pour la fabrication des complexes métalliques polymères moulés selon les revendications 1 à 10, caractérisé en ce qu'on précondense le complexe métallique monomère obtenu par la réaction du composé métallique (VII) avec les composants phosphine (VIII) selon les revendications 11 ou 20 avec de la phosphine (VIII) qui est éventuellement présente en excès, pendant ou après sa fabrication et en ce qu'on précondense un aminosilane de formule (X) ainsi que, le cas échéant, un ou plusieurs composés de formule (XI), indépendamment les uns des autres en présence d'une quantité d'eau qui n'est pas suffisante pour une hydrolyse totale, pendant un laps de temps compris entre 5 minutes et 48 heures entre la température ambiante et 200 °C, en ce qu'on rassemble ensuite les différents composés précondensés et en ce qu'on réalise ensuite l'hydrolyse et la polycondensation complètes ainsi que le reste du traitement selon la revendication 11, après addition d'une quantité d'eau telle qu'on est en présence d'au moins la quantité d'eau stoechiométriquement nécessaire pour une hydrolyse complète et, le cas échéant, d'un autre solvant.

**22.** Procédé pour la fabrication des complexes métalliques polymères moulés selon les revendications 1 à 10, caractérisé en ce qu'on fait réagir, mais sans précondensation, un composé métallique (VII) exempt d'eau avec un composant phosphine (VIII) selon les revendications 11 à 20 et en ce qu'on précondense, indépendamment de ladite réaction, un aminosilane (X) ainsi que, le cas échéant, ou plusieurs composés (XI), sans utilisation ou en utilisant un solvant, en présence d'une quantité d'eau qui n'est pas suffisante pour une hydrolyse complète, pendant un laps de temps compris entre 5 min. et 48 heures entre la température ambiante et 200 °C, en ce qu'on rassemble le produit de réaction non précondensé contenant le métal avec les deux produits de précondensation et en ce qu'on réalise ensuite l'hydrolyse et la polycondensation complètes ainsi que le reste du traitement selon la revendication 11, après addition d'une autre quantité d'eau et, le cas échéant, de solvant supplémentaire, de telle manière qu'on est en présence d'au moins la quantité d'eau stoechiométriquement nécessaire pour une hydrolyse et une polycondensation complètes.

**23.** Procédé pour la fabrication des complexes métalliques polymères moulés selon les revendications 1 à 10, caractérisé en ce qu'on fait réagir un composé métallique (VII) contenant de l'eau ou exempt d'eau, de préférence dans un solvant polaire, avec une phosphine (VIII) en présence d'un aminosilane (X) ainsi que, le cas échéant, d'un ou de plusieurs composés (XI) pendant un laps de temps compris entre 1 minute et 48 heures, selon les revendications 11 ou 20, en ce qu'on ajoute à la solution, sous agitation, une quantité d'eau au moins suffisante pour l'hydrolyse et la condensation complètes et en ce qu'on procède ensuite selon la revendication 11.

**24.** Procédé selon la revendication 23, caractérisé en ce qu'on précondense pendant ou après la réaction des composants selon la revendication 16.

**25.** Procédé pour la fabrication des complexes métalliques polymères moulés, pour lesquels, dans la formule (VI), X = H ou le métal lié par complexation a une valence zéro, selon les revendications 1 à 10, caractérisé en ce que, selon les revendications 11 à 24, on soumet les complexes métalliques monomères préparés en premier lieu, avant ou après la précondensation éventuellement réalisée, à un traitement avec un agent de réduction, le cas échéant à une température élevée, pendant un laps de temps compris entre 1 minute et 48 heures et en ce qu'on réalise ensuite l'hydrolyse ultérieure, la polycondensation et le traitement selon la revendication 11.

**26.** Procédé pour la fabrication des complexes métalliques polymères moulés, pour lesquels, dans la formule (VI), X = H ou le métal lié par complexation a une valence zéro, selon les revendications 1 à 10, caractérisé en ce que, selon les revendications 11 à 24, on hydrolyse et on polycondense en moulant d'abord les complexes métalliques monomères préparés en premier lieu et en ce qu'on les soumet, avant ou après au moins une des étapes de traitement prévues selon la revendication 11, à l'état de susDension dans l'eau ou un solvant, à un traitement de réduction selon la revendication 25, le cas échéant sous une surpression.

**27.** Procédé pour le traitement ultérieur des complexes métalliques polymères moulés obtenus selon une ou plusieurs des revendications 11 à 26, mais non séchés, caractérisé en ce que le complexe encore humide de solvant ou d'eau, est soumis, en présence d'eau ainsi que, le cas échéant, en présence d'un solvant soluble dans l'eau ou du dernier liquide utilisé au cours de la préparation, en tant que tel ou sous forme de vapeur, à un traitement thermique pendant un laps de temps d'une heure à une semaine entre 50 et 300 °C, de préférence entre 100 et 200 °C, le cas échéant sous une surpression, le cas échéant en réalisant simultanément un traitement de réduction selon la revendication 26, de préférence dans une atmosphère de d'hydrogène et/ou avec du borohydrure de sodium.

**28.** Procédé selon la revendication 27, caractérisé en ce que le traitement ultérieur est réalisé en présence d'un catalyseur de condensation ou d'hydrolyse acide, basique ou contenant un métal.

**29.** Utilisation des complexes métalliques polymères moulés selon les revendications 1 à 10 comme catalyseurs pour la réalisation de réactions d'hydroformylation, d'hydratation, d'oligomérisation, de carbonylation, d'hydrosylilation, de carboxyméthylation, d'isomérisation ainsi que pour des réactions de CO ou de $CO_2$ avec du $H_2$.

**30.** Utilisation selon la revendication 29, caractérisée en ce que les complexes métalliques polymères moulés sont utilisés en suspension ou dans un lit solide ou dans un lit fluidisé pour les réactions en phase liquide ou gazeuse.